# FASCICULE DE BREVET EUROPEEN

(11) **EP 2 874 619 B1**
(45) Date de publication et mention de la délivrance du brevet: **04.01.2023**
(21) Numéro de dépôt: 13726205.1
(22) Date de dépôt: 30.05.2013
(51) Int. Cl.: A61K 9/00, A61P 17/00, A61P 17/02, A61P 17/06, A61P 29/00, A61P 37/06, A61P 37/08, A61K 31/402, A61K 47/06, A61K 47/10, A61K 47/26, A61K 9/107, A61K 31/40

(54) **COMPOSITIONS PHARMACEUTIQUES TOPIQUES DE TYPE ÉMULSION H/E CONTENANT UN RÉTINOÏDE**
TOPISCHE PHARMAZEUTISCHE ZUSAMMENSETZUNGEN AUS O/W-EMULSIONEN MIT EINEM RETINOID
O/W-EMULSION-TYPE TOPICAL PHARMACEUTICAL COMPOSITIONS CONTAINING A RETINOID

(30) Priorité: 01.06.2012 US 201261654694 P; 01.06.2012 FR 1255091
(43) Date de publication de la demande: 27.05.2015
(73) Titulaire: Galderma Research & Development, 06410 Biot (FR)
(72) Inventeur: DUPRAT, Agnès, F-06250 Mougins (FR); MALLARD, Claire, F-06250 Mougins (FR)
(74) Mandataire: Nederlandsch Octrooibureau
(86) Numéro de dépôt international: PCT/EP2013/061199
(87) Numéro de publication internationale: WO 2013/178758

(56) Documents cités:
- EP-A1- 0 780 115
- WO-A1-2006/066978
- WO-A1-2007/039057
- WO-A1-2008/148968

## Description

L'invention se rapporte à une composition pharmaceutique topique comprenant en tant qu'actif pharmaceutique, un rétinoïde dans un milieu physiologiquement acceptable, à son procédé de préparation, et à son utilisation en dermatologie.

Dans le domaine de la dermatologie et de la formulation des compositions pharmaceutiques, l'homme du métier est amené à réaliser des compositions qui doivent être stables physiquement et chimiquement. Elles doivent également permettre de libérer l'actif et de favoriser sa pénétration à travers les couches cutanées afin d'en améliorer son efficacité.

La couche cornée ou stratum corneum est la partie la plus superficielle de l'épiderme. Classiquement assimilé à un mur de «brique et de mortier », il est constitué de cellules mortes, les cornéocytes, enchâssées dans des lipides intercornéocytaires. Le stratum corneum repose sur l'épiderme qui est la première couche viable de la peau. Il contient de nombreux types cellulaires et est avasculaire. Enfin le derme est constitué d'un faible nombre de cellules, de nombreuses protéines assurant le soutien du tissu, et d'un réseau vasculaire. Si le stratum corneum est principalement de nature lipophile en raison de la présence des lipides intercornéocytaires, l'épiderme et le derme sont principalement de nature hydrophile, la présence de vaisseaux dans le derme assurant de plus la clairance de ce compartiment.

Il est communément admis que les mécanismes de pénétration cutanée et de perméation sont dépendants d'une part des coefficients de partition des composés entre le véhicule appliqué et les différents compartiments de la peau (stratum corneum, épiderme puis derme vasculaire), et d'autre part du coefficient de diffusion de ces composés dans chaque couche de la peau. Selon leurs caractéristiques physicochimiques, notamment leur caractère lipophile, certains composés présentent une affinité et un coefficient de diffusion élevés dans le stratum, ils sont donc stockés dans le stratum, et dans une moindre mesure dans l'épiderme. En revanche, leur partition dans le derme, vascularisé, de nature plus hydrophile que l'épiderme ou le stratum, sera faible. Dans ce cas, les couches supérieures de la peau, et notamment le stratum, constituent alors un véritable réservoir permettant l'accumulation de l'actif pharmaceutique. Typiquement, la cinétique de pénétration de tels composés présente au cours du temps une augmentation de la quantité d'actif dans le stratum, et dans une moindre mesure dans l'épiderme suivie d'un plateau pendant lequel cette quantité ne varie plus. L'état d'équilibre entre la pénétration de l'actif dans le compartiment et sa clairance est alors atteint.

Dans certains cas, il est important de pouvoir moduler cette cinétique typique de sorte à ce que l'actif pharmaceutique puisse pénétrer dans la peau de façon faible, prolongée et contrôlée. Ce type de pénétration et d'accumulation dans la peau peut alors s'apparenter à une cinétique d'ordre zéro, en référence aux cinétiques de diffusion et de perméation transcutanée d'ordre zéro, linéaires, observées pour certaines préparations galéniques appliquées sur membrane ou sur peau.

Les molécules actives dont on souhaite le passage jusqu'à leur cible sont rarement isolées, elles se trouvent le plus souvent au sein d'une formulation plus ou moins complexe qui, selon les cas, peut être une crème, une pommade, une lotion, une poudre ou un gel.

Après la phase de contact entre la molécule et la surface de la peau, la substance active devra quitter son véhicule pour pénétrer au sein de la couche cornée, avec plus ou moins de facilité.

Généralement, les compositions pharmaceutiques topiques telles que les gels, les crèmes, les lotions, les solutions libèrent le ou les principes actifs par diffusion directement proportionnelle au gradient de concentration au sein de la composition.

En d'autres termes, après application sur la peau le ou les principes actifs sont libérés relativement immédiatement puis la cinétique de libération tend vers zéro pour obtenir un plateau. Le ou les principes actifs ne sont plus alors absorbés sur la peau.

Afin d'obtenir une cinétique de libération indépendante du gradient de concentration, il existe plusieurs types de formulations topiques tels que :
- La formulation de solutions supersaturées, c'est-à-dire des compositions dans lesquelles le ou les principes actifs sont en solution très concentré(s),
- La réalisation de systèmes transdermiques (patches) dans lesquels la libération de ou des principes actifs est contrôlée par une membrane, qui est perméable et généralement adhésive, directement en contact avec la peau.

Les solutions supersaturées sont bien souvent instables et ne permettent pas d'obtenir une bonne stabilité physique de la composition qui peut être un gel, une crème, une lotion ou une pommade.

Les systèmes transdermiques sont par contre plus stables mais présentent l'inconvénient d'avoir une mise en œuvre plus complexe avec une surface d'application limitée par la taille du patch.

Le problème que se propose de résoudre ici la présente invention, est donc de concevoir une composition pharmaceutique de type émulsion huile dans eau, stable physiquement et chimiquement, comprenant au moins un principe actif, tel qu'un ou plusieurs rétinoïdes, ayant un profil de libération non conventionnel. Elle doit également présenter une facilité d'utilisation et une cosméticité acceptable pour une application sur toutes les zones du corps pouvant être touchées par la pathologie.

Il existe donc le besoin de disposer d'une composition pharmaceutique topique permettant de répondre à un ou plusieurs des aspects suivants :
- d'obtenir une bonne stabilité physique sans risque de cristallisation du ou des principes actifs,
- de permettre des applications sans limitation de surface et bien tolérées,
- d'obtenir une pénétration progressive du ou des principes actifs au niveau de sa cible.

La Demanderesse souhaite donc améliorer ces paramètres par la présente invention.

Par stabilité chimique, on entend notamment une stabilité du principe actif.

Par stabilité physique, on entend notamment une absence de cristallisation ou précipitation de l'actif, ou encore une absence de séparation de phase ou d'une modification de sa couleur au sein de la composition.

Une composition stable physiquement selon l'invention est, par conséquent, une composition ne présentant aucune modification d'aspect macroscopique (séparation de phase, changement de couleur d'aspect, etc..) ni microscopique (recristallisation des actifs) après stockage aux températures de 25°C, 4°C et 40°C, pendant 1 mois, 2 mois, 3 mois et 6 mois.

Une composition stable chimiquement selon l'invention est, par conséquent, une composition dans laquelle la teneur en principe actif reste stable après six mois à température ambiante (TA) et à 40 °C. Une teneur stable en principe actif signifie selon l'invention que la teneur présente très peu de variation par rapport à la teneur initiale, c'est-à-dire que la variation de teneur en principe actif au temps T ne doit pas être inférieure à 90% et plus particulièrement à 95% de la teneur initiale à T0.

D'autres paramètres sont également à prendre en compte par l'homme de l'art pour le choix des ingrédients d'une composition pharmaceutique. En effet, la composition pharmaceutique utilisable selon l'invention en tant que médicament, devra également être formulée en accord avec la pathologie à traiter.

A titre d'exemple non limitatif, une composition destinée à traiter l'acné, se devra d'être d'aspect cosmétique non gras, alors qu'une composition destinée à traiter les ichtyoses, les états ichtyosiformes, l'hyperkératose palmoplantaire ou le psoriasis devra être émolliente, hydratante, et pourra être plus riche en corps gras, tout en évitant l'aspect gras non cosmétique.

Dans la présente invention, la demanderesse a montré de façon surprenante, qu'il était possible d'obtenir une composition de type émulsion huile dans eau, contenant un rétinoïde, stable physiquement et chimiquement, ayant une cinétique de pénétration de l'actif d'ordre zéro.

On entend par composition ayant une cinétique de pénétration d'ordre zéro, une composition, qui une fois appliquée sur la surface de la peau, a un profil de pénétration progressif et contrôlé de l'actif au niveau de sa cible.

Par exemple non limitatif, la cible du rétinoïde se trouve plus particulièrement au niveau de l'épiderme pour le traitement de l'acné, les ichtyoses, les états ichtyosiformes, la kératose palmoplantaire et le psoriasis.

Par forme solubilisée de l'actif, on entend une dispersion de l'actif à l'état moléculaire dans un liquide, aucune cristallisation de l'actif n'étant visible à l'œil nu, ni même au microscope optique en polarisation croisée.

L'acide 3"-tert-butyl-4'-(2-hydroxyethoxy)-4"-pyrrolidin-1-yl[1 ,1';3',1"]terphenyl-4-carboxylique (appelé Composé A dans le reste du texte) appartient à la famille des rétinoïdes qui sont des agonistes du récepteur RAR gamma. Les récepteurs RAR gamma étant situés dans l'épiderme, il est important que la libération du rétinoïde s'effectue dans cette partie de la peau pour avoir une efficacité clinique. Le profil de libération de ce type de molécule dans la peau est très important pour atteindre le site d'activité et d'obtenir une efficacité optimale.

Connaissant les caractéristiques physico-chimiques de l'actif, la Demanderesse a dû faire face à un certain nombre de contraintes de mise en œuvre du Composé A :
- le Composé A est peu soluble dans les solvants habituellement utilisés dans les phases grasses des émulsions topiques.
- le Composé A se dégrade chimiquement dans nombreux de ses solvants.
- le Composé A se dégrade chimiquement en présence de nombreux émulsionnants.

Il n'est donc pas évident d'avoir une bonne stabilité chimique du Composé A lorsqu'il est solubilisé ou en présence d'émulsionnants.

Afin de réaliser une émulsion huile dans eau (H/E) selon l'invention des études de préformulation ont été réalisées afin de mettre en évidence les excipients permettant une bonne solubilisation ainsi qu'une bonne stabilité de l'actif.

### Stabilité du Composé A dans ses principaux solvants huileux (déterminée par HPLC)

| **Excipients** | | **Composé A** | **Résultats stabilité** |
|---|---|---|---|
| **Nom commercial** | **Nom INCI** | **%** | |
| Miglyol 812N | Caprylic/ capric triglycérides | 0,005% | Stable/ Ok 6Mois 40°C |
| Huile d'amande douce | Prunus Amygdalus Dulcis (Sweet Almond) Oil | 0,005% | Stable/ Ok 6Mois 40°C |
| Capryol 90 | Propylène glycol monocaprylate | 0,05% | Stable/ Ok 6Mois 40°C |
| Arlacel 83V Pharma | Sorbitan Sesquioleate | 0,05% | Stable/ Ok 6Mois 40°C |
| Lauroglycol FCC | Propylène glycol laurate | 0,05% | Stable/ Ok 6Mois 40°C |
| Arlamol E | PPG-15 stearyl ether | 0,05% | Stable/ Ok 6Mois 40°C |
| Phenoxetol | Phenoxyethanol | 0,05% | Stable/ Ok 6Mois 40°C |
| Labrafil M1944CS | Apricot Kernel Oil PEG-6 Ester | 0,05% | Stable/ Ok 6Mois 40°C |
| Dipropylène Glycol Care | Dipropylène glycol | 0,05% | Instable |
| Brij 30 | Laureth-4 | 0,05% | Instable |
| Alcool benzylique | Benzyl Alcohol | 0,05% | Instable |
| Eutanol G | Octyldodecanol | 0,05% | Instable |
| Myritol PC | Propylène glycol dicaprylate/ dicaprate | 0,05% | Instable |
| Arlasolve DMI | Dimethyl Isosorbide | 0,05% | Instable |

Les limites fixées pour une bonne stabilité sont 95%-105% en pourcentage relatif par rapport au T0.

Ces études de stabilité du Composé A dans ses principaux solvants montrent que le Composé A se dégrade chimiquement dans nombreux de ses solvants (instabilité chimique). Les résultats de cette étude, nous ont permis de sélectionner le solvant principal de l'actif et les huiles co-solvantes parmis les solvants présentant des bons résultats de stabilité, ceci dans l'objectif de développer des émusions H/E dans lesquelles l'actif est solubilisé dans la phase huileuse.
- **(3)** Stabilité du Composé A dans des mélanges d'excipients (solvant/ tensio-actifs) déterminée par HPLC :

Des études de stabilité du Composé A solubilisé dans des huiles solvantes dans lesquelles il est stable, comme démontré précédemment, en présence de surfactants ont été réalisées :

| **Mélange excipients** | | **Composé A** | **Résultats stabilité** |
|---|---|---|---|
| **Nom commercial** | **Nom INCI** | **%** | |
| Cremophor EL/ Labrafil M1944CS | PEG-35 Castor Oil/ Apricot Kernel Oil PEG-6 Ester | 0,05% | Instable |
| Tween 80/ Arlamol E | Polysorbate-80/ PPG-15 | 0,05% | Instable |
| | stearyl ether | | |
| Cremophor EL/ Lauroglycol FCC | PEG-35 Castor Oil/ Propylène glycol laurate | 0,05% | Instable |
| Tween 80/ Hexylene glycol | Polysorbate-80/ Hexylene glycol | 0,2% | Instable |
| Cremophor EL/ Labrafil M1944CS | PEG-35 Castor Oil/ Apricot Kernel Oil PEG-6 Ester | 0,2% | Instable |
| Tween 80/ Lauroglycol FCC | Polysorbate-80/ Propylène glycol laurate | 0,2% | Instable |
| Arlacel 165/ Lauroglycol FCC | Glyceryl Stearate | 0,05% | Instable |
| | PEG-100 Stearate/ Propylène glycol laurate | | |
| GlucateSS-Glucamate SSE-20/ Arlamol E | Methyl Glucose Sesquistearate- PEG-20 | 0,05% | Instable |
| | Methyl Glucose Sesquistearate/ PPG-15 stearyl ether | | |

Les limites fixées pour une bonne stabilité sont 95%-105% en pourcentage relatif par rapport au T0.

Ces études ont montré que le Composé A se dégrade chimiquement en présence de nombreux surfactants classiquement utilisés dans les émulsion de type huile dans eau (H/E).

Le but de la présente invention est le développement de compositions pharmaceutiques contenant un rétinoïde sous la forme d'émulsions de type H/E (Huile dans Eau) à base d'émulsionnants de type esters de sucrose dans lesquelles l'actif est solubilisé dans la phase grasse. Dans ces compositions pharmaceutiques, le rétinoïde présente une bonne stabilité physique et chimique. Dans ces compositions, le rétinoïde pénètre de façon progressive au niveau de sa cible.

La Demanderesse a découvert que lorsque le Composé A est solubilisé avec des solvants et co-solvants dans lesquels il est stable et lorsque on utilise des esters de sucrose comme agents émulsionnants, les compositions selon l'invention sont stables au moins 6 mois à température ambiante et à +40°C.

La présente invention a pour objet une composition pharmaceutique topique de type émulsion huile dans eau comprenant une phase lipophile, un ou plusieurs surfactants et se présente sous la forme d'un pré-concentré qui après dilution avec une phase aqueuse permet d'obtenir des émulsions micro et submicroniques.

Les constituants de la phase grasse doivent être choisis de manière à être solvant de l'actif et peuvent être associés à d'autres huiles ou corps gras afin de préparer une composition ayant des propriétés souhaitées.

La composition selon l'invention concerne donc une composition topique de type émulsion huile dans eau, comprenant :
- une phase grasse contenant au moins un principe actif choisi parmi les rétinoïdes, au moins un solvant principal de l'actif, une ou plusieurs huiles co-solvantes, et une huile minérale,
- une phase aqueuse contenant au moins un tensioactif de la famille des esters de sucrose, au moins un polyol et de l'eau purifiée.

Par rétinoïde, on entend au sens de la présente invention, un rétinoïde hydrophobe tel que les composés protégés dans la demande de brevet WO2006/066978 tel que l'acide 3"-tert-butyl-4'-(2-hydroxyethoxy)-4"-pyrrolidin-1-yl[1,1';3',1"]terphenyl-4-carboxylique.

La composition selon l'invention concerne donc une composition topique de type émulsion huile dans eau, comprenant :
- une phase grasse contenant l'acide 3"-tert-butyl-4'-(2-hydroxyethoxy)-4"-pyrrolidin-1-yl[1,1';3',1"]terphenyl-4-carboxylique, au moins un solvant principal de l'actif, une ou plusieurs huiles co-solvantes, et une huile minérale,
- une phase aqueuse contenant au moins un tensioactif de la famille des esters de sucrose, au moins un polyol et au moins 5% en poids d'eau.

Selon un mode préféré, la composition selon l'invention concerne une composition topique de type émulsion huile dans eau, comprenant :
- une phase grasse contenant au moins un principe actif choisi parmi les rétinoïdes, au moins un solvant principal de l'actif, une ou plusieurs huiles co-solvantes, et une huile minérale,
- une phase aqueuse contenant au moins un tensioactif de la famille des esters de sucrose, au moins un polyol, au moins un gélifiant et au moins 5% en poids d'eau.

Par gélifiant, on entend un composé polymère apte à conférer à la composition la texture d'un gel. Il peut s'agir de gélifiants d'origine végétale, de gommes, de pectines, de celluloses et de ses dérivés, de gélifiants d'origine microbiologiques tel que la gomme xanthane, de gélifiants d'origine synthétique.

Selon un mode préféré, la composition selon l'invention concerne donc une composition pharmaceutique de type émulsion huile dans eau comprenant une phase grasse contenant l'acide 3"-tert-butyl-4'-(2-hydroxyethoxy)-4"-pyrrolidin-1-yl[1,1';3',1"]terphenyl-4-carboxylique (composé A), en tant que principe actif, le phénoxyéthanol en tant que solvant principal du composé A, une ou plusieurs huiles co-solvantes choisie parmi caprylic/capric triglycérides, l'huile d'amande douce. apricot kernel oil PEG-6 Ester, l'huile de coprah raffiné, et une ou plusieurs huiles minérales, une phase aqueuse comprenant au moins un tensioactif de la famille des esters de sucrose, au moins un polyol et de l'eau purifiée.

Dans un mode particulièrement préféré, la composition se présente sous forme d'émulsion et comprend :
- une phase grasse, comprenant entre 0,00001 et 1 % d'au moins un rétinoïde,
- une phase aqueuse comprenant de 0.1 à 15% de esters de sucrose, de 1 à 40% de polyol, de 0.005 à 10% de gélifiant hydrophile et au moins 5% d'eau,
- de 0 à 15% d'un ou plusieurs additifs.

Selon un mode particulier de l'invention, la composition est composée :
- une phase grasse comprenant de 0.00001 % à 1 % d'au moins un rétinoïde; de 0.1 à 10% de solvant principal du rétinoïde ; de 0.5 à 60% d'huiles co-solvantes du rétinoïde ; de 0.5 à 20% d'huiles minérales ; de 0.1 à 10% de 0.5 à 3 % d'épaississant de phase grasse ; de 0 à 20% d'huile de silicone ;
- une phase aqueuse comprenant de 0.1 à 15% d'émulsionnants de la famille des esters sucrose ; de 1 à 40% de 4 à 10% de polyol ; de 0,005 à 10 % de gélifiant de la phase aqueuse ;
- de 0.01 à 5% de système conservateur ;
- et de 0 à 15% de 0.1 à 10% d'additifs.

Selon un mode particulier de l'invention, la composition plus particulièrement adaptée au traitement de l'acné est composée :
- d'une phase grasse comprenant de 0.00001 % à 1 % d'acide 3"-tert-butyl-4'-(2-hydroxyethoxy)-4"-pyrrolidin-1-yl[1,1';3',1"]terphenyl-4-carboxylique (composé A); de 0.2 à 5% de solvant principal du composé A ; de 0.5 à 60% d'huiles co-solvantes du composé A; de 0.5 à 20% d'huiles minérales ; de 0.1 à 10% d'épaississant de phase grasse ; de 0 à 20% d'huile de silicone ;
- d'une phase aqueuse comprenant de 0.1 à 15% d'émulsionnants de la famille des esters sucrose ; de 1 à 40% de polyol ; de 0,005 à 10 % de gélifiant de la phase aqueuse ;
- de 0.01 à 5% de système conservateur ;
- et de 0.001 à 15% d'additifs.

Dans le cas de compositions pharmaceutiques adaptées au traitement de l'acné, de manière préférée la phase grasse est composée de :
- 0,00001 à 1 % d'au moins un rétinoïde
- 1 % de phénoxyéthanol ;
- 1 ,980% d'alcool cétearylique ;
- 3% d'huiles minérales ;
- et 15 à 22% d'huiles co-solvantes.

Selon un mode plus particulier de l'invention la composition pour le traitement de l'acné est composée:
- d'une phase grasse comprenant de 0.00001 % à 1 % d'acide 3"-tert-butyl-4'-(2-hydroxyéthoxy)-4"-pyrrolidin-1-yl[1,1' ;3',1"]terphényl-4-carboxylique (composé A); de 0.2 à 5% de phénoxyéthanol en tant que solvant principal du composé A, de 0.5 à 60% d'huiles co-solvantes du composé A choisies parmi caprylic/capric triglycérides, l'huile d'amande douce, apricot kernel oil PEG-6 Ester, l'huile de coprah raffiné; de 0.5 à 20% d'huiles minérales; de 0.1 à 10% d'épaississant de phase grasse; de 0 à 20% d'huile de silicone;
- d'une phase aqueuse comprenant de 0.1 à 15% d'émulsionnants de la famille des esters sucrose; de 1 à 40% de polyol; de 0,005 à 10 % de gélifiant de la phase aqueuse;
- de 0.01 à 5% de système conservateur choisi parmi le methylparabène, le propylparabène, l'alcool benzylique et le sorbate de potassium ;
- et de 0.001 à 15% d'additifs.

Selon un mode particulier de l'invention, la composition plus particulièrement adaptée au traitement les ichtyoses, les états ichtyosiformes, l'hyperkératose palmoplantaire ou le psoriasis est composée :
- d'une phase grasse comprenant de 0.00001% à 1% d'acide 3"-tert-butyi-4'-(2-hydroxyethoxy)-4"-pyrrolidin-1-yl[1,1';3',1"]terphenyl-4-carboxylique (composé A); de 0.2 à 5% de solvant du composé A; de 0.5 à 60% d'huiles co-solvantes du composé A ; de 0.5 à 20% d'huiles minérales ; de 0.1 à 10% d'épaississant de phase grasse ; de 0 à 20% d'huile de silicone ;
- d'une phase aqueuse comprenant de 0.1 à 15% d'émulsionnants de la famille des esters de sucrose ; de 1 à 40% de polyol ; de 0,005 à 10 % de gélifiant de la phase aqueuse;
- de 0.01 à 5% de système conservateur;
- et de 0.001 à 15% d'additifs.

Selon un mode de l'invention la composition pour le traitement des ichtyoses, les états ichtyosiformes, l'hyperkératose palmoplantaire ou le psoriasis est composée :
- d'une phase grasse comprenant de 0.00001% à 1% d'acide 3"-tert-butyl-4'-(2-hydroxyéthoxy)-4"-pyrrolidin-lyl[l,l';3',l "]terphényl-4-carboxylique (composé A); de 0.2 à 5% de phénoxyéthanol en tant que solvant principal du composé A, de 0.5 à 60% d'huiles co-solvantes du composé A, choisies parmi caprylic/capric triglycérides, 1'huile d'amande douce, apricot kernel oil PEG-6 Ester, l'huile de coprah raffiné; de 0.5 à 20% d'huiles minérales ; de 0.1 à 10% d'épaississant de phase grasse; de 0 à 20% d'huile de silicone;
- d'une phase aqueuse comprenant de 0.1 à 15% d'émulsionnants de la famille des esters de sucrose; de 1 à 40% de polyol; de 0,005 à 10% de gélifiant de la phase aqueuse;
- de 0.01 à 5% de système conservateur choisi parmi le methylparabène, le propylparabène, l'alcool benzylique, et le sorbate de potassium;
- et de 0.001 à 15% d'additifs.

Le système tensio-actif comprend au moins un tensio-actif principal choisi dans la catégorie des esters de sucroses tels que le sucrose stéarate et le sucrose palmitate. Ce ou ces esters de sucre peuvent être associés à tout autre tensio-actif pouvant jouer le rôle de co-tensio-actifs.

La phase aqueuse peut contenir de manière non limitative un ou plusieurs polyols, un ou plusieurs gélifiants de phase hydrophile, différents types d'additifs. Les % sont exprimés en poids par rapport au poids total de la composition sauf mention particulière.

Dans l'invention, les compositions contiennent de l'acide 3"-tert-butyl-4'-(2-hydroxyethoxy)-4"-pyrrolidin-1-yl[1,1';3',1"]terphenyl-4-carboxylique, appelé Composé A à des concentrations allant de 0.00001 % à 1% et préférentiellement de 0.0001 à 0.1 % en poids par rapport à poids total de la composition.

Comme indiqué précédemment, la phase aqueuse contient au moins un tensioactif de la famille des esters de sucrose.

Dans l'invention, les compositions contiennent des tensio-actifs de type esters de sucrose qui ont été choisis par la Demanderesse pour leur bonne compatibilité avec le Composé A, mais aussi par ce qu'ils permettent la réalisation d'émulsions très fines présentant des textures très douces et une excellente tolérance cutanée.

Les esters de sucrose sont des tensioactifs non ioniques comprenant un groupe hydrophile constitué par la partie sucrose et un groupe lipophile constitué par un acide gras. Le sucrose ayant généralement un total de 8 groupes hydroxyles, il est donc possible d'obtenir des esters de sucrose allant d'un « mono » ester de sucrose à un « octa » ester de sucrose. Les « esters de sucroses » ou « sucrose esters » sont des esters d'acides gras et de sucrose, le sucrose étant un disaccharide composé de glucose et de fructose. Par ester de sucrose, on entend à titre d'exemple non limitatif, le sucrose myristate vendu sous le nom de Surfhope C-1416 fourni par GATTEFOSSE, du sucrose palmitate vendu sous le nom de Surfhope C-1615, Surfhope C-1616 et Surfhope SE Pharma D-1616 fourni par GATTEFOSSE mais aussi vendu sous le nom de Sisterna PS750-C et fourni par UNIPEX, du sucrose stéarate vendu sous le nom de Surfhope C-1811 , Surfhope SE Pharma D-1816, Surfhope C-1815 et Surfhope C-1816 fourni par GATTEFOSSE mais aussi vendu sous le nom de Sisterna SP50-C, Sisterna SP70-C et fourni par UNIPEX.

Dans un mode préféré selon l'invention, la composition comprend le palmitate de sucrose, ou le stéarate de sucrose ou leur mélange à une concentration allant de 0.1 à 15% et préférentiellement de 0.5 à 5% en poids par rapport au poids total de la composition.

La phase grasse selon l'invention doit être choisie de manière à contenir au moins un solvant principal du composé A, une ou plusieurs huiles co-solvantes, et une huile minérale.

Par solvant principal, on entend un liquide qui a la propriété de dissoudre, de diluer ou d'extraire d'autres substances sans provoquer de modification chimique de ces substances et sans lui-même se modifier.

Selon l'invention, un solvant principal est un tel liquide, dans lequel les rétinoïdes (plus préférentiellement le composé A) présentent une solubilité, à température ambiante et pression atmosphérique, supérieure ou égale à 0,1 % en poids.

Préférentiellement, le solvant principal est présent à une concentration allant de 0.1 à 10%.

Dans le cas où l'actif de la composition selon l'invention est le Composé A, le solvant principal peut être, par exemple, le phénoxyethanol vendu sous le nom de phenoxetol par Clariant présent à une concentration allant de 0.2 à 5% et préférentiellement de 0.5 à 2%.

Par co-solvant, on entend une substance ayant un rôle de solvant en association avec une autre substance.

La phase grasse de l'invention comprend également :
- une ou plusieurs huiles co-solvantes, et de façon préférentielle les huiles co-solvantes suivantes : Caprylic/ capric triglycérides (Miglyol 812N) fourni par IMCD, Prunus Amygdalus Dulcis (Sweet Almond) oil (Huile d'amande douce) fournie par SICTIA, Propylène glycol monocaprylate (Capryol 90) fourni par GATTEFOSSE, Propylène glycol laurate (Lauroglycol FCC) fourni par GATTEFOSSE, , PPG-15 stearyl ether (Arlamol PS15E-LQ) fourni par CRODA, Sorbitan Sesquioleate (Arlacel 83VPharma) fourni par CRODA, Apricot Kernel Oil PEG-6 Ester, (Labrafil M1944CS) fourni par GATTEFOSSE, l'huile de coprah raffinée fourni par OLVEA à des taux allant de 0.5 à 60% et préférentiellement de 10 à 20% pour l'indication acné et entre 20 et 40% pour les indications ichtyose, hyperkératose palmoplantaires et psoriasis.
- une ou plusieurs huiles minérales, comme les huiles de paraffine de différentes viscosités comme le Marcol 152, le Marcol 52 ou le Primol 352 vendu par UNIVAR, à des taux allant de 0,5% à 20% et préférentiellement de 2 à 6%.

D'autres huiles ou corps gras peuvent être ajoutés à la phase grasse de la composition de manière variée par l'homme du métier afin de préparer une composition ayant les propriétés souhaitées, par exemple en consistance ou en texture.

La composition selon l'invention peut contenir par exemple également :
- Une ou plusieurs huiles de silicone améliorant les propriétés de la formule à l'application, comme la Cyclomethicone (St-Cyclomethicone 5NF) ou la Dimethicone (Q7 9120 silicon fluid de viscosité de 20 cst à 12500 cst de Dow Corning) entre 0 et 20% et préférentiellement entre 0 et 6%.
- un ou plusieurs épaississants de phase grasse de type alcool gras comme l'alcool cétylique (Crodacol C70 fourni par CRODA/ Kolliwax CA fourni par BASF), l'alcool cétearylique (Crodacol 1618 fourni par CRODA, Tego Alkanol 1618 fourni par EVONIK mais aussi Kolliwax CSA 50), l'alcool stéarylique (Crodacol S95 fourni par CRODA, Kolliwax SA fourni par BASF Tego Alkanol 18 fourni par EVONIK) mais aussi l'alcool behenique (Nacol 22-98 fourni par SASOL mais aussi Behenyl Alcohol 65 80 fourni par NIKKO CHEMS) ou de type cire de Carnauba fourni par BAERLOCHER mais aussi la cire d'abeille vendu sous le nom de CERABEIL BLANCHIE DAB fourni par UNIVAR entre 0.1 et 10% et préférentiellement entre 0.5 et 6%.

Ainsi, la phase grasse de l'émulsion selon l'invention peut être présente à une teneur comprise entre 1 et 95% en poids par rapport au poids total de la composition, de préférence entre 5 et 85%, plus préférentiellement entre 15 et 50% en poids par rapport au poids total de la composition.

Les bonnes stabilités chimiques et physiques de la composition selon l'invention sont obtenues notamment par le choix des tensioactifs. Ainsi, la composition selon l'invention comprend également au moins un tensio-actif principal choisi dans la catégorie des esters de sucrose.

La composition selon l'invention étant une émulsion huile dans eau, elle comporte une phase aqueuse contenant au moins 5% d'eau par rapport au poids total de la composition, de préférence entre 5 et 90%.

Dans un mode préféré selon l'invention, la phase aqueuse contient également un polyol (au minimum un triol), de préférence sélectionné parmi la glycérine, la diglycérine ou le sorbitol (Neosorb fourni par ROQUETTE, Parteck SI fourni par Merck mais aussi Sorbitol USP Powder fourni par LIPO CHEMICALS) et dont la quantité est comprise entre 1 et 40% en poids par rapport au poids total de la composition et préférentiellement entre 4 et 10% pour l'indication acné et entre 10 et 25% pour les indications ichtyose, hyperkératose palmoplantaires et psoriasis.

Dans un mode préféré, la composition selon l'invention contient de la glycérine à une teneur comprise entre 1 et 20% et une proportion d'eau comprise entre 5% et 90%.

Dans un mode de réalisation, la composition selon l'invention comprend également un ou plusieurs gélifiants de phase hydrophiles. A titre d'exemple non limitatif de gélifiants pouvant entrer dans les compositions selon l'invention, on peut citer l'Acrylates/C10-30 Alkyl Acrylate Crosspolymer vendu sous le nom de Pemulen TR-1 ou Pemulen TR-2 par la société Lubrizol, les carbomers vendus sous le nom d'Ultrez 20^{®}, d'Ultrez 10^{®}, de Carbopol 1382^{®} ou de Carbopol ETD2020NF^{®}, de Carbopol 981 ou encore Carbopol 980 par la Société Lubrizol, les polysaccharides avec à titre d'exemples non limitatifs la gomme de xanthane telle que le Xantural180^{®} vendu par la société Kelco ou la Satiaxane UCX 911 vendue par Cargill, l'alcool polyvinylique tel que Polyvinil alcohol 40-88 vendue par Merck, la gellan gum vendue sous le nom de Kelcogel par la société Kelco, la gomme guar, la cellulose et ses dérivés tel que la microcrystalline cellulose et carboxymethyl cellulose de sodium vendues sous le nom d'Avicel CL-611 par la société FMC Biopolymer, l'hydroxypropylmethylcellulose en particulier le produit vendu sous le nom de Methocel E4M premium par la société Dow Chemical ou l'hydroxyéthylcellulose, en particulier, le produit vendu sous le nom de Natrosol HHX 250^{®} par la société Ashland, la famille des aluminium magnésium silicates tel que le Veegum K vendu par la société Vanderbilt, la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%), la famille des amidons modifiés tels que l'amidon de pomme de terre modifié vendu sous le nom de Structure Solanace ou bien leurs mélanges .et les gélifiants de la famille des polyacrylamides tels que le mélange Sodium acryloyldimethyltaurate copolymer /isohexadecane /polysorbate 80 vendu sous le nom Sepineo P600^{®} (ou de Simulgel 600 PHA^{®}) par la société Seppic, le mélange polyacrylamide / isoparaffine C13-14 /laureth-7 comme, par exemple, celui vendu sous le nom de Sepigel 305 par la société Seppic, la famille des carraghénanes en particulier réparties sous quatre grandes familles : κ, λ, β, ω tel que les Viscarin^{®} et les Gelcarin^{®} commercialisés par la société IMCD.

Préférentiellement, seront utilisés les gélifiants de type polyacrylamides comme le Sodium acryloyldimethyltaurate copolymer /isohexadecane /polysorbate 80 pour leur bonne compatibilité avec le principe actif et leur bonne capacité à stabiliser les émulsions, aux concentrations préférentielles allant de 0,005 à 10 % et plus préférentiellement de 0.5% à 4%.

Dans un mode de réalisation particulièrement préféré, la composition selon l'invention comprend également un ou plusieurs des conservateurs, tels que le methylparabène, le propylparabène, le chlorure de benzalkonium, le phénoxyethanol vendu sous le nom de phenoxetol par Clariant, l'alcool benzylique vendu sous le nom d'alcool benzylique par Merck, le benzoate de sodium vendu sous le nom de Probenz SP par Unipex, le sorbate de potassium vendu sous le nom de Sorbate de potassium par VWR, l'acide benzoique vendu sous le nom Acide benzoïque par VWR, le 2-Bromo-2-Nitropropane-1,3-Diol vendu sous le nom de Bronopol par Jan Dekker International, la chlorhexidine vendu sous le nom de Chlorexidine digluconate 20% solution par Arnaud Pharmacie, le chlorocrésol et ses dérivés, l'alcool éthylique et la diazolidinylurée. Ces conservateurs peuvent être utilisés seul ou en association afin de protéger efficacement les formules contre toute contamination bactérienne.

Par conservateur, on entend on appelle conservateur toute substance capable de s'opposer aux altérations d'origine chimique ou microbiologique d'un produit.

Les conservateurs utilisés préférentiellement dans l'invention sont les methylparabène, propylparabène, alcool benzylique, phénoxyethanol et sorbate de potassium. Ils peuvent être utilisés de 0.01% à 5% et préférentiellement de 0.05 à 2%.

La composition selon l'invention peut également comprendre des additifs usuellement utilisés dans le domaine pharmaceutique et cosmétique permettant de conférer à la dite préparation des propriétés spécifiques. L'homme de l'art adaptera le choix de ces additifs en fonction de l'effet attendu.

Parmi les additifs on peut citer en exemple et à titre non limitatif, pris seuls ou combinés :
- des agents chélatants comme l'EDTA (éthylène diamine tétra-acide acétique) et ses dérivés ou sels, la dihydroglycerine, les acides citriques et tartriques, la gluconolactone vendu sous le nom D-(+)-glucono-delta-lactone par Jungbunzlauer ou des mélanges de ceux-ci.
- des antioxydants tel que la vitamine E et ses dérivés, comme le DL alpha Tocopherol ou l'acetate de tocopherol de Roche, la vitamine C et ses dérivés, comme l'Ascorbyl Palmitate de Roche, le Butylhydroxy toluene vendu sous le nom de Nipanox BHT par Clariant,
- des agents apaisants et/ou anti-irritants tels que le PPG-12/SMDI copolymer vendu par la société Bertek pharmaceuticals sous le nom commercial de Polyolprepolymer-2, l'acide glycyrrhétinique ou ses dérivés comme par exemple l'Enoxolone vendu par la société BASF, l'acide hyaluronique tel quel ou sous sa forme hyaluronate de sodium vendu sous le nom commercial de Hyal. Na PWD PH 15-51-45 par la société Contipro, l'allantoïne vendue sous le nom de Ronacare allantoine par Merck.
- tout autre additif usuellement utilisé dans le domaine pharmaceutique et cosmétique permettant de conférer à la dite préparation des propriétés spécifiques.

Les additifs seront présents au sein de la composition selon l'invention dans des proportions allant de 0 à 15%, et préférentiellement de 0.1 à 10% en poids total de la composition.

La technologie utilisée permet l'obtention d'émulsions très fines, les globules huileux obtenus ont une taille majoritairement inférieure à 5µm. Le procédé de fabrication est constitué de deux principales étapes :
- La première étape consiste en la formation d'une phase intermédiaire « gel », il s'agit d'une émulsion H/E contenant une large proportion de phase grasse et ayant un aspect gel transparent constituée de l'actif, la phase grasse, l'ester de sucrose, le polyol et l'eau.
- La deuxième étape consiste en la dilution spontanée de la phase intermédiaire « gel » dans la phase aqueuse afin d'obtenir une émulsion huile dans eau très fine. La viscosité finale de l'émulsion est dépendante du paramètre de dilution. La stabilité de l'émulsion peut être renforcée par l'ajout d'un agent gélifiant.

Le procédé de fabrication est particulièrement sensible. La vitesse et la température d'introduction de certains ingrédients ont un impact important sur la stabilité physique des émulsions de notre invention.

La présente invention a également pour objet le procédé de fabrication de composition selon l'invention.

Le procédé de fabrication général des émulsions à base d'«esters de sucrose » est décrit ci-dessous :

### ∘ A) Préparation de la phase «esters de sucrose »

Solubiliser les « esters de sucrose » dans une partie de l'eau purifiée et une partie du polyol.

### ∘ B) Préparation de la phase grasse/ active

Solubiliser le rétinoide dans le solvant principal de l'actif, la ou les huiles co-solvantes, le ou les épaississants de phase grasse et l'huile minérale. Chauffer la phase grasse lors de sa préparation peut être préconisé afin d'obtenir de meilleurs résultats.

### ∘ C) Préparation de la phase intermédiaire « gel »

La phase intermédiaire « gel » est obtenue en mélangeant la phase «esters de sucrose » et la phase grasse/ active,

### ∘ D) Préparation de la phase aqueuse

Dans la quantité d'eau restante ajouter le reste du polyol ainsi que les autres additifs hydrosolubles.

### ∘ E) Obtention de la crème

L'émulsion finale est obtenue par dilution de la phase intermédiaire avec la phase aqueuse. L'homme du métier saura adapter ce procédé fin d'y ajouter les ingrédients ou additifs voulus.

Le procédé de fabrication particulièrement préféré des émulsions à base d' «esters de sucrose » est décrit ci-dessous :

### ∘ A) Préparation de la phase «esters de sucrose »

Solubiliser les « esters de sucrose » dans une partie de l'eau purifiée et une partie de la glycérine.

### ∘ B) Préparation de la phase grasse/ active

Solubiliser le Composé A dans l'ensemble des ingrédients lipophiles sauf l'alcool benzylique, la cyclomethicone qui seront ajoutés en fin de fabrication.

Chauffer la phase grasse lors de sa préparation peut être préconisé afin d'obtenir de meilleurs résultats.

### ∘ C) Préparation de la phase intermédiaire « gel »

La phase intermédiaire « gel » est obtenue en mélangeant la phase «esters de sucrose » et la phase grasse/ active,

### ∘ D) Préparation de la phase aqueuse

Dans la quantité d'eau restante ajouter le reste de glycérine ainsi que les autres additifs hydrosolubles.

L'acide hyaluronique tel quel ou sous sa forme de sel peut être ajouté à cette phase.

### ∘ E) Obtention de la crème

L'émulsion finale est obtenue par dilution de la phase intermédiaire avec la phase aqueuse et par l'ajout de l'alcool benzylique, cyclométhicone et le gélifiant de type polyacrylamide.

La présente invention a également pour objet une composition pharmaceutique pour son utilisation dans le traitement des pathologies suivantes :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) les affections d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tels que le lymphome T.

De préférence, la composition est une composition pharmaceutique pour son utilisation dans le traitement des affections dermatologiques liées à un désordre de la kératinisation portant sur la différenciation et sur la prolifération cellulaire, les troubles de la kératinisation et les affections dermatologiques avec une composante immuno-allergique inflammatoire, avec ou sans trouble de la prolifération cellulaire.

Plus préférentiellement, la composition selon l'invention est une composition pharmaceutique pour son utilisation dans le traitement de l'acné, les ichtyoses, les états ichtyosiformes, l'hyperkératose palmoplantaire ou le psoriasis.

Conformément à ces modes de réalisation préférés, la composition selon l'invention comprend préférentiellement le Composé A.

En particulier, la composition est de préférence une composition pharmaceutique pour son utilisation dans le traitement de l'acné qui comprend ;
- une phase grasse comprenant de 0.00001% à 1% d'acide 3"-tert-butyl-4'-(2-hydroxyéthoxy)-4"-pyrrolidin-1-yl[1 ,1';3',1"]terphényl-4-carboxylique (composé A); de 0.2 à 5% de solvant principal du composé A ; de 0.5 à 60% d'huiles co-solvantes du composé A; de 0.5 à 20% d'huiles minérales ; de 0.1 à 10% d'épaississant de phase grasse ; de 0 à 20% d'huile de silicone ;
- une phase aqueuse comprenant de 0.1 à 15% d'émulsionnants de la famille des esters sucrose ; de 1 à 40% de polyol ; de 0,005 à 10 % de gélifiant de la phase aqueuse ;
- de 0.01 à 5% de système conservateur ;
- et de 0.001 à 15% d'additifs.

De manière alternative, la composition est de préférence une composition pharmaceutique pour son utilisation dans le traitement des ichtyoses, des états ichtyosiformes, de l'hyperkératose palmoplantaire ou du psoriasis composée :
- d'une phase grasse comprenant de 0.00001% à 1% d'acide 3"-tert-butyl-4'-(2-hydroxyethoxy)-4"-pyrrolidin-1-yl[1 ,1';3',1"]terphenyl-4-carboxylique (composé A); de 0.2 à 5% de solvant du composé A; de 0.5 à 60% d'huiles co-solvantes du composé A ; de 0.5 à 20% d'huiles minérales ; de 0.1 à 10% d'épaississant de phase grasse ; de 0 à 20% d'huile de silicone ;
- d'une phase aqueuse comprenant de 0.1 à 15% d'émulsionnants de la famille des esters de sucrose ; de 1 à 40% de polyol ; de 0,005 à 10 % de gélifiant de la phase aqueuse;
- de 0.01 à 5% de système conservateur ;
- et de 0.001 à 15% d'additifs.

### I- Exemples de formulations :

Dans les exemples suivants, les formules réalisées sont caractérisées à T0. La stabilité physique et chimique des formulations est réalisée après conservation à température ambiante (TA) et +40°C après T+1Mois et/ou T+2Mois ou T+3Mois ou T+6Mois. Le matériel et les méthodes utilisés pour ces caractérisations est décrit ci-dessous.

### -Dosage chimique du Composé A:

- Matériel: HPLC
- Expression des résultats: le titre de l'actif est exprimé en % relatif par rapport au % initial effectué à T0. Les limites fixées pour une bonne stabilité sont 95%-105%

### -Observation macroscopique:

- L'observation macroscopique permet de garantir l'intégrité physique des produits à T0 et après stabilité.

### -Observation microscopique:

- L'observation microscopique permet d'évaluer la bonne solubilisation du composé A dès T0, la non recristallisation au cours du temps ainsi que la taille des globules de la phase huileuse.

### - pH:

- Méthode : Mesures du pH effectuées à température ambiante après stabilisation 24h dans une enceinte à 25°C de tous les échantillons.

### -Viscosité:

- La mesure de la viscosité permet d'évaluer la consistance des formules réalisées.
- Matériel: Brookfield RV DVII + Pro
- Méthode: Mesures effectuées à température ambiante après stabilisation 24h dans une enceinte à 25°C de tous les échantillons. La valeur est lue après 1 minute. Le choix du mobile et de la vitesse seront décrits dans chaque exemple de composition. Les valeurs obtenues sont exprimées en centipoises (Cps)

### -Centrifugation:

- La centrifugation permet d'évaluer la résistance des formules à une contrainte mécanique.
- Méthode: 30 minutes à 5000 rpm
- Un résultat conforme signifie qu'il n'y a ni séparation de phases, ni exudat.

Les exemples suivants montrent de façon non exhaustive des exemples de formulation de la composition selon l'invention ainsi que des résultats de stabilité chimique et physique.

### I-1-Exemples de formules adaptées à l'acné:

Les exemples 1 à 11 peuvent être fabriqués de 0,001% à 0,03% en composé A.

### Exemple 1

| COMPOSITIONS | | Exemple 1 |
|---|---|---|
| Nom Commercial | Nom INCI | % |
| Composé A | NA | 0,010 |
| GLYCERINE | GLYCERIN | 6,950 |
| SURFHOPE SE PHARMA D-1816 | SUCROSE STEARATE | 0,825 |
| SURFHOPE SE PHARMA D-1616 | SUCROSE PALMITATE | 0,825 |
| PHENOXETOL | PHENOXYETHANOL | 1,000 |
| MIGLYOL812N | CAPRYLIC/ CAPRIC TRIGLYCERIDE | 15,000 |
| ALCOOL BENZYLIQUE | BENZYL ALCOHOL | 0,400 |
| SORBATE DE POTASSIUM | POTASSIUM SORBATE | 0,100 |
| SIMULGEL 600PHA | ACRYLAMIDE/ SODIUM ACRYLOYLDIMETHYLTAURATE COPOL YMER ISOHEXADECANE POLYSORBATE 80 | 3,000 |
| RONACARE ALLANTOINE | ALLANTOIN | 0,200 |
| SPEZIOL C16-18 PHARMA | CETYL STEARYL ALCOHOL | 1,980 |
| PRIMOL 352 | MINERAL OIL | 1,750 |
| MARCOL 152 | MINERAL OIL | 1,250 |
| HYALURONATE DE SODIUM | SODIUM HYALURONATE | 1,750 |
| CYCLOMETHICONE 5NF | CYCLOPENTASILOXANE | 4,000 |
| EAU PURIFIEE | NA | QSP 100.000 |

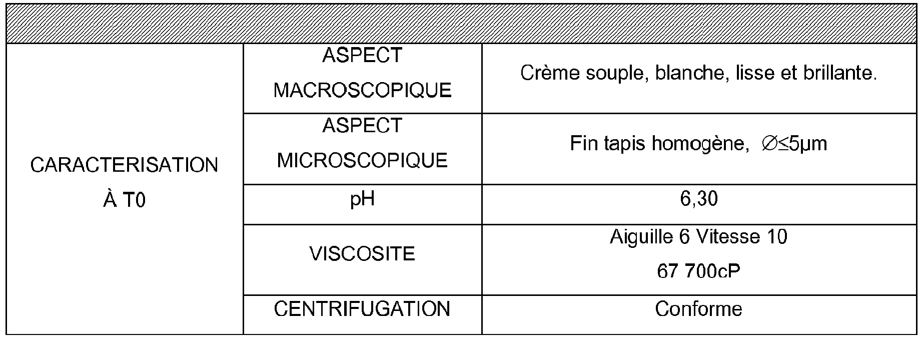

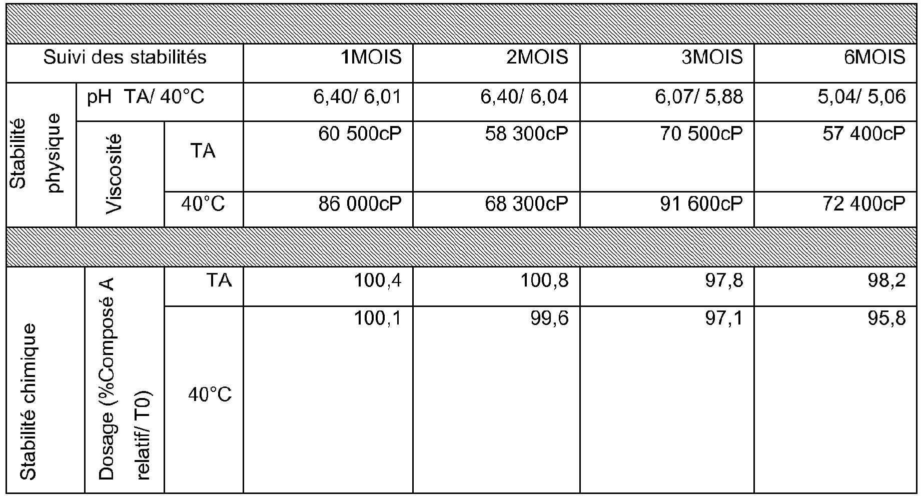

Ces résultats montrent une bonne stabilité physique et chimique de l'actif et de la composition dans son ensemble dans le temps.

### Exemple 2

| COMPOSITIONS | | Exemple 2 |
|---|---|---|
| Nom Commercial | Nom INCI | % |
| Composé A | NA | 0,010 |
| GLYCERINE 4810 VEGETABLE | GLYCERIN | 6,950 |
| SURFHOPE SE PHARMA D-1816 | SUCROSE STEARATE | 0,825 |
| SURFHOPE SE PHARMA D-1616 | SUCROSE PALMITATE | 0,825 |
| PHENOXETOL | PHENOXYETHANOL | 1,000 |
| MIGLYOL812N | CAPRYLIC/ CAPRIC TRIGLYCERIDE | 15,000 |
| NIPAGIN M | METHYLPARABEN | 0,200 |
| NIPASOL M | PROPYLPARABEN | 0,100 |
| SIMULGEL 600PHA | ACRYLAMIDE/ SODIUM ACRYLOYLDIMETHYLTAURATE COPOL YMER ISOHEXADECANE POLYSORBATE 80 | 1,200 |
| RONACARE ALLANTOIN | ALLANTOIN | 0,200 |
| SPEZIOL C16-18 PHARMA | CETYL STEARYL ALCOHOL | 1,980 |
| PRIMOL 352 | MINERAL OIL | 1,750 |
| MARCOL 125 | MINERAL OIL | 1,250 |
| CYCLOMETHICONE 5NF | CYCLOPENTASILOXANE | 4,000 |
| EAU PURIFIEE | NA | QSP 100.000 |

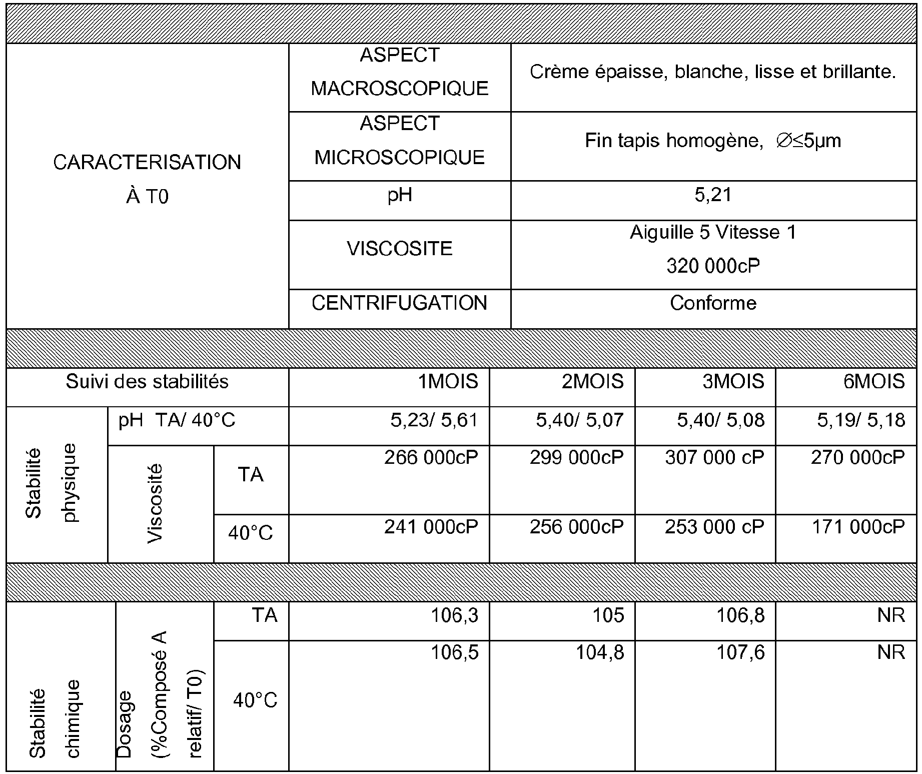

Ces résultats montrent une bonne stabilité physique et chimique de l'actif et de la composition dans son ensemble dans le temps.

### Exemple 3

| COMPOSITIONS | | Exemple 3 |
|---|---|---|
| Nom Commercial | Nom INCI | % |
| Composé A | NA | 0,010 |
| GLYCERINE | GLYCERIN | 6,950 |
| SURFHOPE SE PHARMA D-1816 | SUCROSE STEARATE | 0,825 |
| SURFHOPE SE PHARMA D-1616 | SUCROSE PALMITATE | 0,825 |
| PHENOXETOL | PHENOXYETHANOL | 1,000 |
| MIGLYOL812N | CAPRYLIC/ CAPRIC TRIGLYCERIDE | 15,000 |
| NIPAGIN M | METHYLPARABEN | 0,200 |
| NIPASOL M | PROPYLP ARABEN | 0,100 |
| SIMULGEL 600PHA | ACRYLAMIDE/ SODIUM ACRYLOYLDIMETHYLTAURATE COPOL YMER ISOHEXADECANE POLYSORBATE 80 | 1,200 |
| RONACARE ALLANTOIN | ALLANTOIN | 0,200 |
| SPEZIOL C16-18 | CETYL STEARYL ALCOHOL | 1,980 |
| PRIMOL 352 | MINERAL OIL | 1,750 |
| MARCOL 152 | MINERAL OIL | 1,250 |
| HYALURONATE DE SODIUM | SODIUM HYALURONATE | 0,200 |
| CYCLOMETHICONE 5NF | CYCLOPENTASILOXANE | 4,000 |
| EAU PURIFIEE | NA | QSP 100.000 |

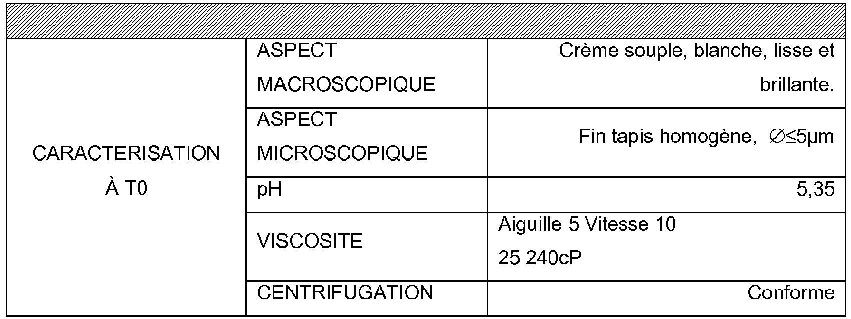

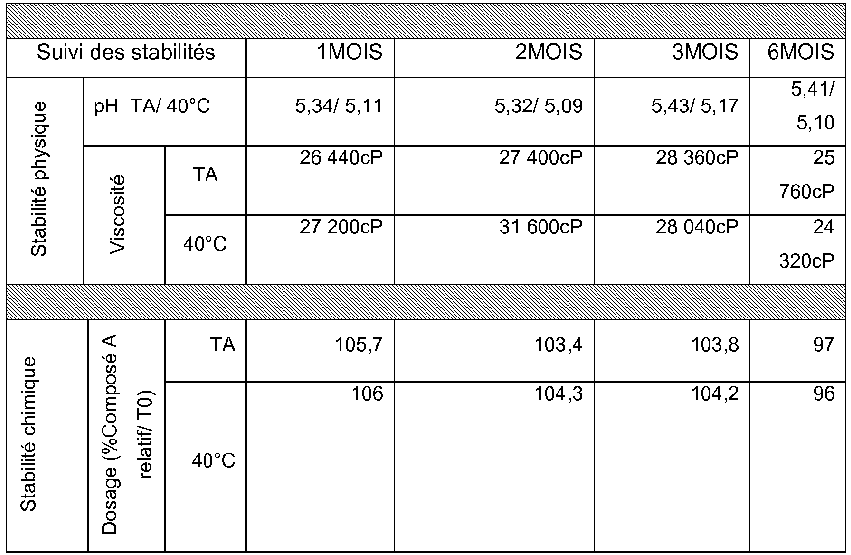

Ces résultats montrent une bonne stabilité physique et chimique de l'actif et de la composition dans son ensemble dans le temps.

### Exemple 4

| COMPOSITIONS | | Exemple 4 |
|---|---|---|
| Nom Commercial | Nom INCI | % |
| Composé A | NA | 0,010 |
| GLYCERINE | GLYCERIN | 6,950 |
| SURFHOPE SE PHARMA D-1816 | SUCROSE STEARATE | 0,825 |
| SURFHOPE SE PHARMA D-1616 | SUCROSE PALMITATE | 0,825 |
| PHENOXETOL | PHENOXYETHANOL | 1,000 |
| MIGLYOL812N | CAPRYLIC/ CAPRIC TRIGLYCERIDE | 15,000 |
| NIPAGIN M | METHYLPARABEN | 0,200 |
| NIPASOL M | PROPYLPARABEN | 0,100 |
| SIMULGEL 600PHA | ACRYLAMIDE/ SODIUM ACRYLOYLDIMETHYLTAURATE COPOL YMER ISOHEXADECANE POLYSORBATE 80 | 1,200 |
| RONACARE ALLANTOINE | ALLANTOIN | 0,200 |
| SPEZIOL C16-18 PHARMA | CETYL STEARYL ALCOHOL | 1,980 |
| PRIMOL 352 | MINERAL OIL | 1,750 |
| MARCOL 152 | MINERAL OIL | 1,250 |
| POLYVINYL ALCOHOL 40-88 | POLYVINYL ALCOHOL | 0,500 |
| CYCLOMETHICONE 5NF | CYCLOPENTASILOXANE | 4,000 |
| EAU PURIFIEE | NA | QSP 100.000 |

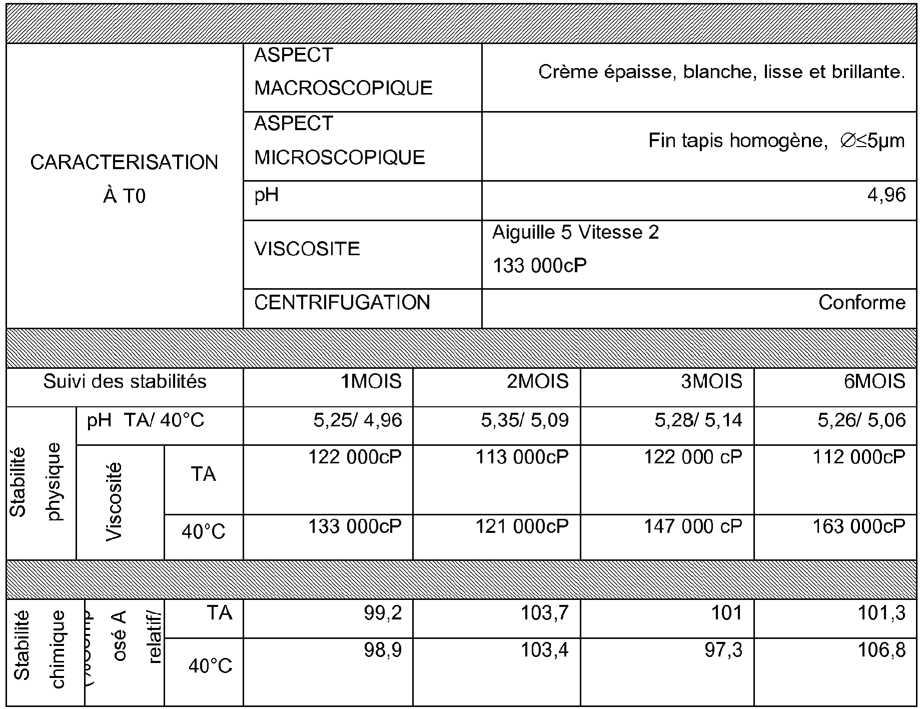

Ces résultats montrent une bonne stabilité physique et chimique de l'actif et de la composition dans son ensemble dans le temps.

### Exemple 5

| COMPOSITIONS | | Exemple 5 |
|---|---|---|
| Nom Commercial | Nom INCI | % |
| Composé A | NA | 0,005 |
| GLYCERINE | GLYCERIN | 6,950 |
| SURFHOPE SE PHARMA D-1816 | SUCROSE STEARATE | 0,825 |
| SURFHOPE SE PHARMA D-1616 | SUCROSE PALMITATE | 0,825 |
| PHENOXETOL | PHENOXYETHANOL | 1,000 |
| MIGLYOL812N | CAPRYLIC/ CAPRIC TRIGLYCERIDE | 15,000 |
| NIPASOL M | PROPYLP ARABEN | 0,100 |
| NIPAGIN M | METHYLPARABEN | 0,200 |
| SIMULGEL 600PHA | ACRYLAMIDE/ SODIUM ACRYLOYLDIMETHYLTAURATE COPOL YMER ISOHEXADECANE POLYSORBATE 80 | 1,200 |
| RONACARE ALLANTOINE | ALLANTOIN | 0,200 |
| SPEZIOL C16-18 PHARMA | CETYL STEARYL ALCOHOL | 1,980 |
| PRIMOL 352 | MINERAL OIL | 1,750 |
| MARCOL 152 | MINERAL OIL | 1,250 |
| HYALURONATE DE SODIUM | SODIUM HYALURONATE | 0,200 |
| CYCLOMETHICONE 5NF | CYCLOPENTASILOXANE | 4,000 |
| EAU PURIFIEE | NA | QSP 100.000 |

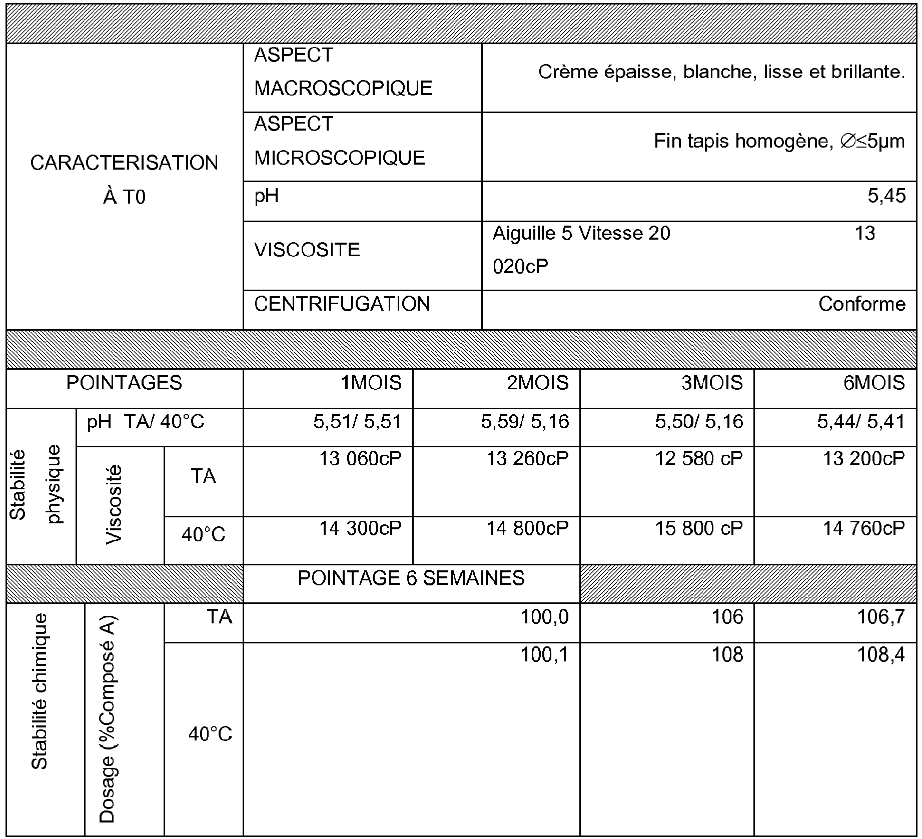

Ces résultats montrent une bonne stabilité physique et chimique de l'actif et de la composition dans son ensemble dans le temps.

### Exemple 6

| COMPOSITIONS | | Exemple 6 |
|---|---|---|
| Nom Commercial | Nom INCI | % |
| Composé A | NA | 0,030 |
| GLYCERINE | GLYCERIN | 6,950 |
| SURFHOPE SE PHARMA D-1816 | SUCROSE STEARATE | 0,825 |
| SURFHOPE SE PHARMA D-1616 | SUCROSE PALMITATE | 0,825 |
| PHENOXETOL | PHENOXYETHANOL | 1,000 |
| MIGLYOL812N | CAPRYLIC/ CAPRIC TRIGLYCERIDE | 15,000 |
| NIPAGIN M | METHYLPARABEN | 0.20 |
| NIPASOL M | PROPYLPARABEN | 0,10 |
| SIMULGEL 600PHA | ACRYLAMIDE/ SODIUM ACRYLOYLDIMETHYLTAURATE COPOL YMER ISOHEXADECANE POLYSORBATE 80 | 1,200 |
| RONACARE ALLANTOINE | ALLANTOIN | 0,200 |
| SPEZIOL C16-18 PHARMA | CETYL STEARYL ALCOHOL | 1,980 |
| PRIMOL 352 | MINERAL OIL | 1.75 |
| MARCOL 152 | MINERAL OIL | 1.25 |
| HYALURONATE DE SODIUM | SODIUM HYALURONATE | 0,200 |
| CYCLOMETHICONE 5NF | CYCLOPENTASILOXANE | 4,000 |
| EAU PURIFIEE | NA | QSP 100.000 |

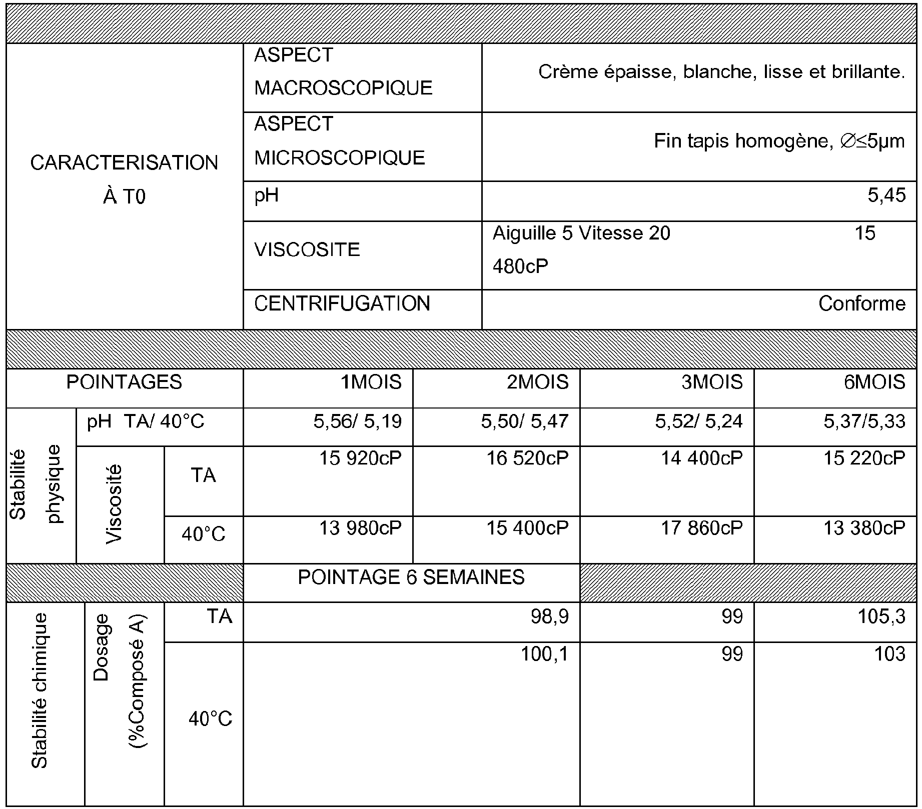

Ces résultats montrent une bonne stabilité physique et chimique de l'actif et de la composition dans son ensemble dans le temps.

### Exemple 7

| COMPOSITIONS | | Exemple |
|---|---|---|
| Nom Commercial | Nom INCI | % |
| Composé A | NA | 0,010 |
| SURFHOPE SE PHARMA D-1816 | SUCROSE STEARATE | 0,500 |
| SURFHOPE SE PHARMA D-1616 | SUCROSE PALMITATE | 0,500 |
| PHENOXETOL | PHENOXYETHANOL | 1,000 |
| MIGLYOL812N | CAPRYLIC/ CAPRIC TRIGLYCERIDE | 7,000 |
| HUILE D'AMANDE DOUCE RAFFINEE | PRUNUS AMYGDALUS DULCIS (SWEET ALMOND) OIL | 3,500 |
| PRIMOL 352 | MINERAL OIL | 2,000 |
| CYCLOMETHICONE 5NF | CYCLOPENTASILOXANE | 2,000 |
| SPEZIOL C16-18 | CETYL STEARYL ALCOHOL | 3,000 |
| TITRIPLEX III | DISODIUM EDTA | 0,100 |
| GLYCERINE | GLYCERIN | 6,000 |
| ACIDE BENZOIQUE | BENZOIC ACID | 0,060 |
| SORBATE DE POTASSIUM | POTASSIUM SORBATE | 0,060 |
| | ACRYLAMIDE/ SODIUM | 3,000 |
| | ACRYLOYLDIMETHYLTAURATE | |
| SIMULGEL 600PHA | COPOL YMER | |
| | ISOHEXADECANE | |
| | POLYSORBATE 80 | |
| RONACARE ALLANTOIN | ALLANTOIN | 0,200 |
| ASCORBYL PALMITATE | ASCORBYL PALMITATE | 0,020 |
| EAU PURIFIEE | NA | QSP 100.000 |

### Exemple 8

| COMPOSITIONS | | Exemple |
|---|---|---|
| Nom Commercial | Nom INCI | % |
| Composé A | NA | 0,010 |
| SURFHOPE SE PHARMA D-1816 | SUCROSE STEARATE | 1,200 |
| SURFHOPE SE PHARMA D-1616 | SUCROSE PALMITATE | 1,200 |
| PHENOXETOL | PHENOXYETHANOL | 0,800 |
| ARLAMOL E | PPG-15 STEARYL ETHER | 10,000 |
| HUILE DE COPRAH RAFFINEE | COCOS NUCIFERA OIL | 2,000 |
| LAUROGLYCOL FCC | PROPYLENE GLYCOL LAURATE | 5,000 |
| MARCOL 152 | MINERAL OIL | 2,000 |
| CYCLOMETHICONE 5NF | CYCLOPENTASILOXANE | 4,000 |
| SPEZIOL C16-18 | CETYL STEARYL ALCOHOL | 2,000 |
| GLYCERINE | GLYCERIN | 6,950 |
| MIGLYOL812N | CAPRYLIC/ CAPRIC TRIGLYCERIDE | 15,000 |
| NIPAGIN M | METHYLPARABEN | 0,200 |
| NIPASOL M | PROPYLP ARABEN | 0,100 |
| | ACRYLAMIDE/ SODIUM | 2,000 |
| | ACRYLOYLDIMETHYLTAURATE | |
| SIMULGEL 600PHA | COPOL YMER | |
| | ISOHEXADECANE | |
| | POLYSORBATE 80 | |
| RONACARE ALLANTOIN | ALLANTOIN | 0,200 |
| TITRIPLEX III | DISODIUM EDTA | 0,100 |
| PRIMOL 352 | MINERAL OIL | 1,750 |
| HYALURONATE DE SODIUM | SODIUM HYALURONATE | 0,200 |
| EAU PURIFIEE | NA | QSP 100.000 |

### Exemple 9

| COMPOSITIONS | | Exemple |
|---|---|---|
| Nom Commercial | Nom INCI | % |
| Composé A | NA | 0,020 |
| SURFHOPE SE PHARMA D-1816 | SUCROSE STEARATE | 2,300 |
| SURFHOPE SE PHARMA D-1615 | SUCROSE PALMITATE | 2,300 |
| PHENOXETOL | PHENOXYETHANOL | 1,000 |
| MIGLYOL812N | CAPRYLIC/ CAPRIC TRIGLYCERIDE | 20,000 |
| Q7-9120 SILICONE FLUID 350 CST | DIMETHICONE | 3,200 |
| SPEZIOL C16-18 | CETYL STEARYL ALCOHOL | 3,000 |
| GLYCERINE | GLYCERIN | 6,950 |
| D-(+)-GLUCONO-DELTA-LACTONE | GLUCONOLACTONE | 0,250 |
| PROBENZ SP | BENZOATE DE SODIUM | 0,200 |
| | ACRYLAMIDE/ SODIUM | 1,200 |
| | ACRYLOYLDIMETHYLTAURATE | |
| SIMULGEL 600PHA | COPOL YMER | |
| | ISOHEXADECANE | |
| | POLYSORBATE 80 | |
| RONACARE ALLANTOIN | ALLANTOIN | 0,200 |
| PRIMOL 352 | MINERAL OIL | 1,750 |
| HYALURONATE DE SODIUM | SODIUM HYALURONATE | 0,200 |
| EAU PURIFIEE | NA | QSP 100.000 |

### Exemple 10

| COMPOSITIONS | | Exemple |
|---|---|---|
| Nom Commercial | Nom INCI | % |
| Composé A | NA | 0,005 |
| SURFHOPE SE PHARMA D-1816 | SUCROSE STEARATE | 2,000 |
| SURFHOPE SE PHARMA D-1615 | SUCROSE PALMITATE | 2,000 |
| LABRAFIL M1944CS | APRICOT KERNEL OIL PEG-6 ESTER | 2,800 |
| ARLAMOL E | PPG-15 STEARYL ETHER | 6,750 |
| LAUROGLYCOL FCC | PROPYLENE GLYCOL LAURATE | 2,200 |
| PRIMOL 352 | MINERAL OIL | 2,000 |
| Q7-9120 SILICONE FLUID 350 CST | DIMETHICONE | 2,000 |
| SPEZIOL C16-18 | CETYL STEARYL ALCOHOL | 2,000 |
| PHENOXETOL | PHENOXYETHANOL | 0,800 |
| NIPAGIN M | METHYLPARABEN | 0,200 |
| NIPASOL M | PROPYLP ARABEN | 0,100 |
| | ACRYLAMIDE/ SODIUM | 2,200 |
| | ACRYLOYLDIMETHYLTAURATE | |
| SIMULGEL 600PHA | COPOL YMER | |
| | ISOHEXADECANE | |
| | POLYSORBATE 80 | |
| RONACARE ALLANTOIN | ALLANTOIN | 0,200 |
| HYALURONATE DE SODIUM | SODIUM HYALURONATE | 0,200 |
| EAU PURIFIEE | NA | QSP 100.000 |

### Exemple 11

| COMPOSITIONS | | Exemple |
|---|---|---|
| Nom Commercial | Nom INCI | % |
| Composé A | NA | 0,030 |
| SURFHOPE SE PHARMA D-1815 | SUCROSE STEARATE | 3,100 |
| SURFHOPE SE PHARMA D-1616 | SUCROSE PALMITATE | 3,100 |
| ARLAMOL PS15E-LQ | PPG-15 STEARYL ETHER | 18,000 |
| HUILE D'AMANDE DOUCE RAFFINEE | PRUNUS AMYGDALUS DULCIS (SWEET ALMOND) OIL | 2,000 |
| PRIMOL 352 | MINERAL OIL | 2,000 |
| MARCOL 152 | MINERAL OIL | 3,000 |
| CYCLOMETHICONE 5NF | CYCLOPENTASILOXANE | 4,000 |
| Q7-9120 SILICONE FLUID 350 CST | DIMETHICONE | 2,000 |
| GLYCERINE | GLYCERIN | 6,950 |
| PHENOXETOL | PHENOXYETHANOL | 1,800 |
| NIPAGIN M | METHYLPARABEN | 0,200 |
| NIPASOL M | PROPYLPARABEN | 0,100 |
| | ACRYLAMIDE/ SODIUM | 1,000 |
| | ACRYLOYLDIMETHYLTAURATE | |
| SIMULGEL 600PHA | COPOL YMER | |
| | ISOHEXADECANE | |
| | POLYSORBATE 80 | |
| RONACARE ALLANTOIN | ALLANTOIN | 0,200 |
| SPEZIOL C16-18 | CETYL STEARYL ALCOHOL | 2,000 |
| ASCORBYL PALMITATE | ASCORBYL PALMITATE | 0,020 |
| HYALURONATE DE SODIUM | SODIUM HYALURONATE | 0,200 |
| EAU PURIFIEE | NA | QSP 100.000 |

### I-2- Exemples de formules adaptées à les ichtyoses, les états ichtyosiformes, l'hyperkératose palmoplantaire ou le psoriasis:

### Exemple 12

| COMPOSITIONS | | Exemple 7 |
|---|---|---|
| Nom Commercial | Nom INCI | % |
| Composé A | NA | 0.01 |
| SURFHOPE SE PHARMA D-1816 | SUCROSE STEARATE | 1.50 |
| SURFHOPE SE PHARMA D-1616 | SUCROSE PALMITATE | 1.50 |
| GLYCERINE | GLYCERIN | 14.00 |
| MIGLYOL 812 N | CAPRYLIC/ CAPRIC TRIGLYCÉRIDES | 15.00 |
| PRIMOL 352 | PARAFFINUM LIQUIDUM (MINERAL OIL) | 4.50 |
| SPEZIOL C16-18 PHARMA | CETEARYL ALCOHOL | 4.50 |
| HUILE DE COPRAH RAFFINÉE | COCOS NUCIFERA (COCONUT) OIL | 6.00 |
| HUILE D'AMANDE DOUCE RAFFINÉE | PRUNUS AMYGDALUS DULCIS (SWEET ALMOND) OIL | 15.00 |
| PHENOXETOL | PHENOXYETHANOL | 1.00 |
| ALCOOL BENZYLIQUE | BENZYL ALCOHOL | 0.40 |
| SORBATE DE POTASSIUM | POTASSIUM SORBATE | 0.10 |
| ALLANTOINE | ALLANTOIN | 0.20 |
| HYALURONATE DE SODIUM | SODIUM HYALURONATE | 0.20 |
| SIMULGEL 600 PHA | ACRYLAMIDE / SODIUM | 1.00 |
| | ACRYLOYLDIMETHYL TAURATE | |
| | COPOLYMER & ISOHEXADECANE & | |
| | POLYSORBATE 80 | |
| EAU PURIFIEE | AQUA (WATER) | QSP |

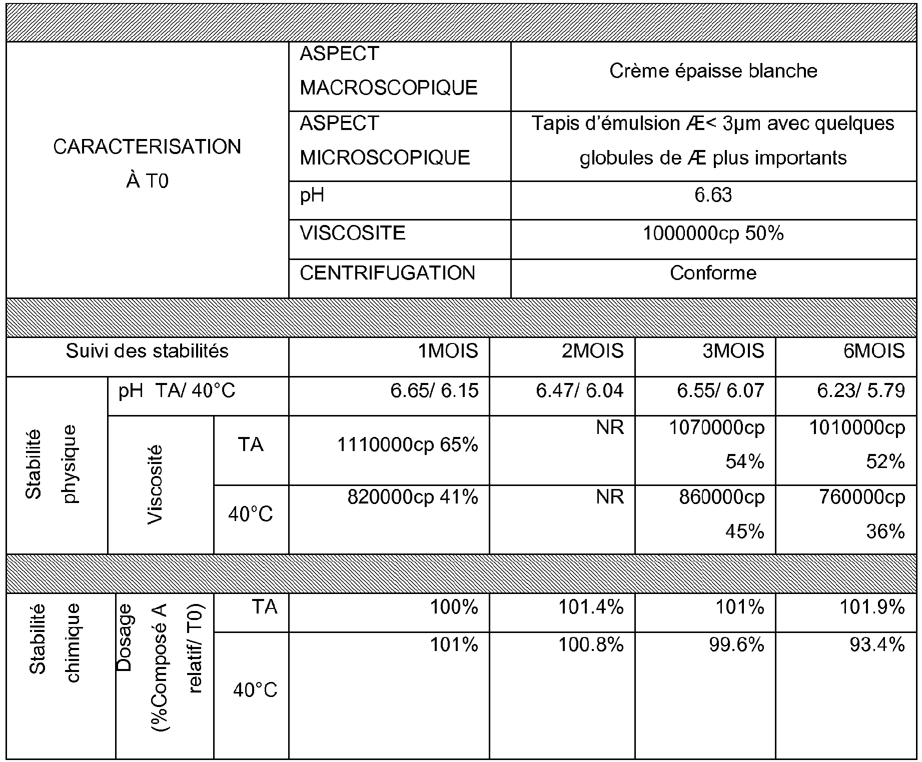

Ces résultats montrent une bonne stabilité physique et chimique de l'actif et de la composition dans son ensemble dans le temps.

### Exemple 13

| COMPOSITIONS | | Exemple 8 |
|---|---|---|
| Nom Commercial | Nom INCI | % |
| Composé A | NA | 0.01 |
| SURFHOPE SE PHARMA D-1816 | SUCROSE STEARATE | 1.25 |
| SURFHOPE SE PHARMA D-1616 | SUCROSE PALMITATE | 1.25 |
| GLYCERINE | GLYCERIN | 12.50 |
| MIGLYOL 812 N | CAPRYLIC/ CAPRIC TRIGLYCÉRIDES | 15.00 |
| PRIMOL 352 | PARAFFINUM LIQUIDUM (MINERAL OIL) | 4.50 |
| SPEZIOL C16-18 PHARMA | CETEARYL ALCOHOL | 2.00 |
| HUILE DE COPRAH RAFFINÉE | COCOS NUCIFERA (COCONUT) OIL | 6.00 |
| HUILE D'AMANDE DOUCE RAFFINÉE | PRUNUS AMYGDALUS DULCIS (SWEET ALMOND) OIL | 10.00 |
| PHENOXETOL | PHENOXYETHANOL | 1.00 |
| ALCOOL BENZYLIQUE | BENZYL ALCOHOL | 0.40 |
| SORBATE DE POTASSIUM | POTASSIUM SORBATE | 0.10 |
| ALLANTOINE | ALLANTOIN | 0.20 |
| HYALURONATE DE SODIUM | SODIUM HYALURONATE | 0.20 |
| SIMULGEL 600 PHA | ACRYLAMIDE / SODIUM | 2.00 |
| | ACRYLOYLDIMETHYL TAURATE | |
| | COPOLYMER & ISOHEXADECANE & | |
| | POLYSORBATE 80 | |
| EAU PURIFIÉE | AQUA (WATER) | QSP |

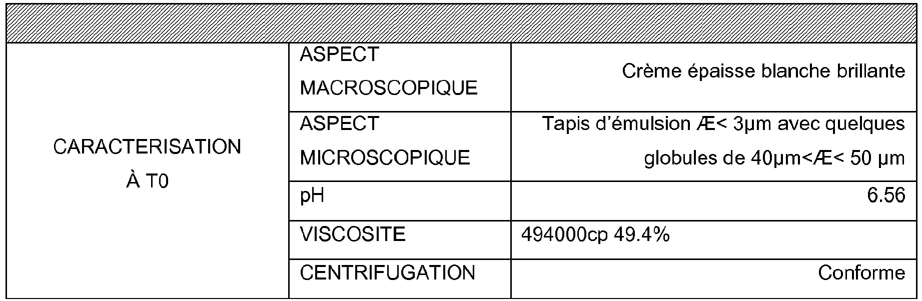

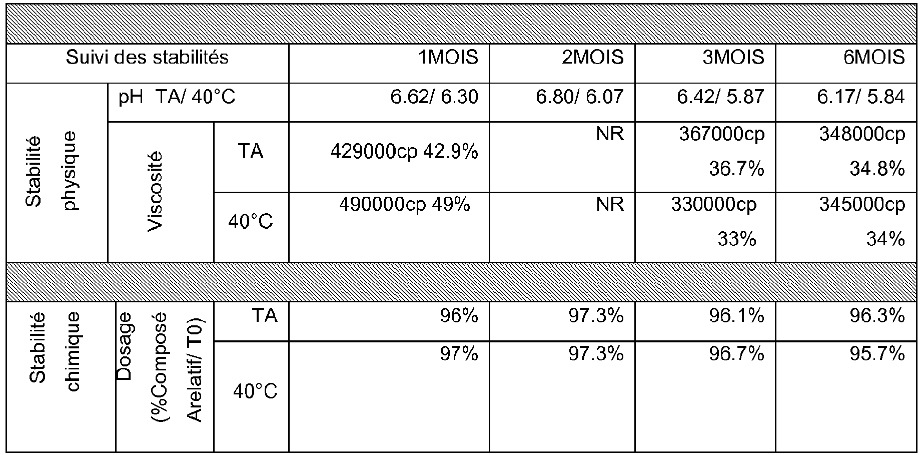

Ces résultats montrent une bonne stabilité physique et chimique de l'actif et de la composition dans son ensemble dans le temps.

### II. Pénétration cutanée sur peau humaine

### II-1-Méthodes d'évaluation in vitro

Les essais in vitro sont développés sur des cellules de diffusion en verre (dites "cellules de Franz") ou en téflon en modes statiques ou dynamiques. Les cellules sont constituées de deux compartiments, un compartiment donneur et un compartiment récepteur séparés par une membrane dont l'orientation peut-être verticale ou horizontale.

Les membranes ont une surface généralement comprises entre 1 et 4°cm², elles peuvent être synthétiques (cellulose, polydimethyl siloxane...) mais les biopsies de peaux animales ou humaines sont préférables.

Des prélèvements de liquide récepteur peuvent être effectués, à temps réguliers sur 24 heures afin de permettre l'établissement de la cinétique de diffusion à travers la peau lorsque cela s'avère nécessaire. A l'issue de maximum 24h d'application, dans le cas des produits pharmaceutiques et cosmétiques, les cellules de Franz sont démontées, la substance étudiée est quantifiée dans les compartiments suivants : reliquat de formulation, stratum corneum, épiderme, derme et liquide récepteur. La balance massique peut dans ce cas être établie ce qui permet de quantifier les doses dites « absorbées » (présentes dans le liquide récepteur), «ayant pénétré » (présentes dans l'épiderme viable et le derme), «non absorbées» (présentes dans le reliquat de formulation et le stratum corneum).

L'étude de la cinétique de pénétration dans la peau permet de donner des informations intéressantes de comportement des formules au cours du temps et permet de caractériser au mieux les compositions formulaires.

Deux types d'études de pénétration cutanée sur peau humaine ex vivo ont donc été réalisées. Dans ces études le composé A a été testé au sein de la composition exemple 1.

### II-2-Etude de pénétration cutanée « temps unique »

Dans cette étude, la formule est appliquée pendant 16h à la surface de la peau humaine issue d'une chirurgie abdominale appliquée dans une cellule de Franz. A la fin de l'application, le composé A est quantifié dans les différents compartiments de la peau : stratum corneum, épiderme, derme et liquide récepteur selon une méthode de bioanalyse validée.

La composition exemple 1 (Emulsion sucroester contenant 100µg/g Composé A) selon l'invention est comparée au gel de référence dont la formule est constitué de : 30% propylène glycol, 67.99% d'éthanol 95-96%, 2% Klucel HF, 0.01% composé A.

La bioanalyse a été réalisée par spectrométrie de masse en tandem par ionisation électrospray positive, et utilisant un appareil Xevo (Waters).

Les conditions de LC/MS/MS mises au point ont permis de détecter jusqu'à 0,1% de la dose appliquée dans chacun des compartiments (dose non absorbée, stratum, épiderme, derme et liquide récepteur).

Les conditions techniques sont données dans le tableau ci-dessous.

Dans ce type d'étude « point unique », les paramètres retenus sont :
a. Le profil de distribution dans les différents compartiments (données qualitatives)
b. La pénétration dans le compartiment épiderme + derme (données numériques)

### a-Profil de distribution dans les différents compartiments

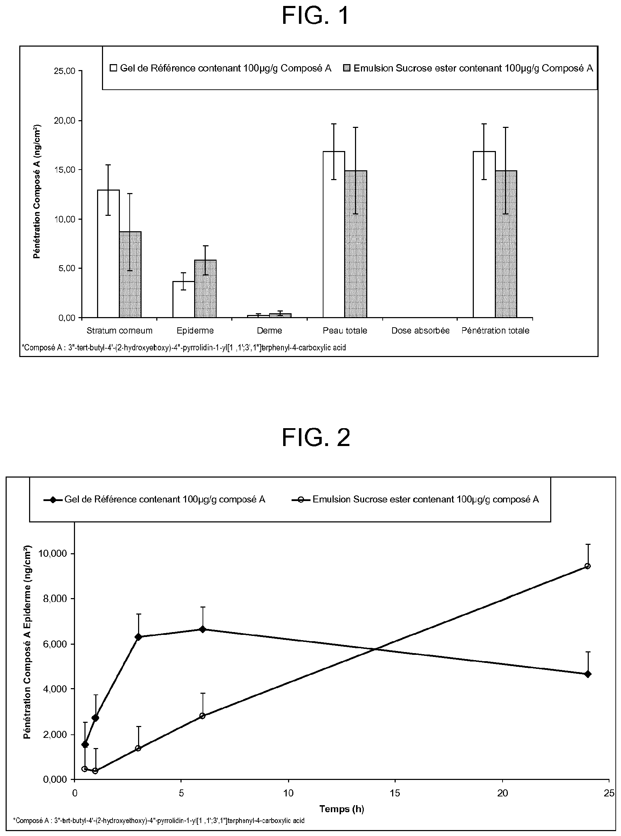

Les résultats obtenus pour le profil de distribution du composé A dans les différents compartiments de la peau sont représentés par la figure 1.

La figure 1 montre que la distribution entre les différents compartiments est du même ordre de grandeur pour les 2 formules évaluées.

Ces données montrent la localisation préférentielle et une accumulation du composé A dans le stratum corneum et de façon moins préférentielle dans l'épiderme.

### b-Valeurs de pénétration dans le compartiment épiderme + derme

Les valeurs de pénétration pour l'émulsion selon l'invention contenant 100µg/g (0.01%) sont comprises entre 0.73ng/cm² et 1,93ng/cm². Ces valeurs sont du même ordre de grandeur que pour le gel de référence.

Nous pouvons donc conclure que le composé A est bien libéré après application sur la peau et pénètre jusque dans l'épiderme.

### II-3-Etude de cinétique de pénétration

Dans ce type d'étude, la pénétration de l'actif est quantifiée dans chaque compartiment de la peau après 0.5h, 1h, 3h 6h et 24h d'application. Une cinétique de pénétration dans chaque compartiment est alors déterminée et caractérisée.

La composition exemple 1 (Emulsion sucroester contenant 100µg/g Composé A) selon l'invention est comparée au gel de référence dont la formule est constituée de : 30% propylène glycol, 67.99% d'éthanol 95-96%, 2% Klucel HF, 0.01% composé A.

Les détails de l'application cutanée sont donnés dans le tableau ci-dessous :

| **Peau** | | **3 donneurs, 2 échantillons par donneur par temps, n=6** |
|---|---|---|
| | Source | Peau humaine abdominale dermatomée de cadavre |
| | Epaisseur | 500µm |
| | Cellules de Franz | 1-2cm² |
| | Fonction Barrière | Evaluée par eau tritiée |

| **Products** | | |
|---|---|---|
| | Gel de référence 100µg/g | Emulsion Exemple 1 100µg/g |
| | Application Formule | ~2mg/cm² |
| | Quantité actif appliquée | Entre 100-200ng/cm² |
| | Temps d'exposition | Jusqu'à 24h |

| **Echantillons repris** | | |
|---|---|---|
| | **Temps d'exposition** | **0.5, 1, 3, 6, 24h** |

| **Analyses** | | **LC/UV et LC/MS** |
|---|---|---|
| | Limite de quantification | 15ng/ml |

La quantité d'actif dans chaque compartiment à chaque temps a été déterminée par LC/UV ou par LC/MS. La méthode de bioanalyse a été validée de sorte à détecter au minimum 0,1% de la dose appliquée dans chaque compartiment.

Dans ce type d'étude, les paramètres retenus sont :
a. Le profil de la cinétique de pénétration dans l'épiderme (données qualitatives)
b. La vitesse initiale de la pénétration dans l'épiderme
c. La quantité maximale pénétrée dans l'épiderme

### a. Profil de la cinétique de pénétration dans l'épiderme (figure 2)

Le profil de la cinétique de pénétration du composé A mis en œuvre avec la formule de référence (gel) et l'émulsion selon l'invention est représenté dans la figure 2. En particulier, la figure 2 représente la quantité du composé A qui pénètre dans l'épiderme en fonction du temps.

La pénétration du composé A obtenue pour la formule de référence (gel) montre une cinétique classique avec une accumulation dans le compartiment au cours des premières heures et ensuite atteinte d'un plateau. En revanche, la cinétique de pénétration observée après application de l'émulsion selon l'invention tend à être progressive et linéaire dans le temps.

Comme vu dans le paragraphe II.2 (Etude de pénétration cutanée « temps unique »), ces deux formules ont des niveaux de pénétration à 16h qui sont du même ordre de grandeur. Cette étude de cinétique montre que même avec des niveaux de pénétration proches dans l'étude de cinétique en temps unique, la formule selon l'invention présente un profil de cinétique de pénétration différent du gel de référence, avec une accumulation progressive du composé A au cours du temps. L'application de l'émulsion selon l'invention a donc permis de modifier de façon importante la cinétique de pénétration du composé A dans la peau. Cette cinétique de pénétration est progressive et linéaire, et s'apparente à une cinétique d'ordre zéro en référence aux cinétiques de diffusion d'un actif à travers une membrane synthétique ou la peau après application d'une préparation galénique.

### b. Vitesse initiale de la cinétique :

Les valeurs de vitesse initiale de la cinétique ou pente sont situées entre 0.45ng/cm²/h et 0.54ng/cm²/h.

### c. Quantité maximale pénétrée dans l'épiderme :

La quantité maximale pénétrée dans l'épiderme est située entre 9.40ng/cm² et 15.50 ng/cm²

Cette étude de pénétration a montré de manière inattendue que les formulations selon l'invention présentaient des caractéristiques particulières de pénétration cutanée avec une cinétique de pénétration du composé A dans l'épiderme tendant à être linéaire, ce qui va permettre de libérer le rétinoïde de façon progressive dans l'épiderme après l'application.

### II-4.Tolérance cutanée

Dans cette étude :
- 10 sujets ont reçu 2 grammes de Gel de référence appliqués sur 1000 cm² pendant 4 semaines.
- 10 sujets ont reçu 2 grammes de Crème A (exemple 1 selon l'invention) appliqués sur 1000 cm² pendant 4 semaines.

Au cours de l'étude, les investigateurs avaient la possibilité de changer de zone d'application en cas d'irritation trop importante.

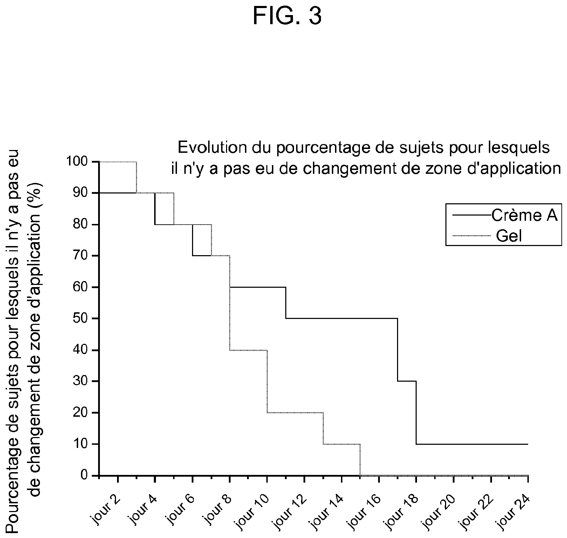

Le graphique de la figure 3 représente le pourcentage de sujets pour lesquels il n'y a eu aucun changement de zone d'application, en fonction du jour d'application.

Par exemple, au jour 5, pour 80% des sujets recevant de la Crème A, il n'y a pas eu besoin de changer de zone d'application. Autrement dit, 20% des sujets ayant reçu de la Crème A ont montré une irritation nécessitant un changement de zone d'application.

### Evolution du pourcentage de sujet pour lesquels il n'y a pas de changement de zone d'application

| | | Crème A 50µg/g 1000 cm² | Gel 50µg/g 1000 cm² | Gel 50µg/g 2000 cm² | Gel 100µg/g 1000 cm² | Gel 25µg/g 1000 cm² |
|---|---|---|---|---|---|---|
| Jour 2 | N(%) | 1 ( 10) | - | - | - | - |
| Jour 4 | N(%) | - | 1 ( 10) | - | - | - |
| Jour 5 | N(%) | 1 ( 10) | - | 2 ( 20) | 1 ( 10) | - |
| Jour 6 | N(%) | - | 1 ( 10) | - | 1 ( 10) | - |
| Jour 7 | N(%) | 1 ( 10) | - | 2 ( 20) | 1 ( 10) | 1 ( 10) |
| Jour 8 | N(%) | - | 1 ( 10) | - | 2 ( 20) | 1 ( 10) |
| Jour 9 | N(%) | 1 ( 10) | 3 ( 30) | 2 ( 20) | 1 ( 10) | - |
| Jour 10 | N(%) | - | - | 1 ( 10) | 1 ( 10) | 1 ( 10) |
| Jour 11 | N(%) | - | 2 ( 20) | - | 1 ( 10) | 1 ( 10) |
| Jour 12 | N(%) | 1 ( 10) | - | 1 ( 10) | - | 1 ( 10) |
| Jour 13 | N(%) | - | - | - | - | - |
| Jour 14 | N(%) | - | 1 ( 10) | 2 ( 20) | 1 ( 10) | 2 ( 20) |
| Jour 16 | N(%) | - | 1 ( 10) | - | 1 ( 10) | - |
| Jour 18 | N(%) | 2 ( 20) | - | - | - | 1 ( 10) |
| Jour 19 | N(%) | 2 ( 20) | - | - | - | - |
| Jour 24 | N(%) | - | - | - | - | 1 ( 10) |
| Aucun changement au cours de l'étude | N(%) | 1 ( 10) | - | - | - | 1 ( 10) |

Ainsi, on constate que l'irritation apparaît plus rapidement chez les sujets qui ont reçu le Gel de référence que chez les sujets qui ont reçu la Crème selon l'invention. C'est à partir du jour 9 que l'on observe une nette différence. La composition selon l'invention est donc mieux tolérée que le gel de référence.

## Revendications

1. Composition pharmaceutique de type émulsion huile dans eau comprenant:
- une phase grasse contenant l'acide 3"-tert-butyl-4'-(2-hydroxyethoxy)-4"-pyrrolidin-1-yl[1,1';3',1"]terphenyl-4-carboxylique (composé A), en tant que principe actif, le phénoxyéthanol en tant que solvant principal du composé A, une ou plusieurs huiles co-solvantes choisie parmi caprylic/capric triglycérides, l'huile d'amande douce, Apricot Kernel Oil PEG-6 Ester, l'huile de coprah raffiné, et une ou plusieurs huiles minérales,
- une phase aqueuse comprenant au moins un tensioactif de la famille des esters de sucrose, au moins un polyol et de l'eau purifiée.

2. Composition selon la revendication 1 **caractérisée en ce que** le tensioactif est choisi parmi le palmitate de sucrose, ou le stéarate de sucrose ou leur mélange.

3. Composition selon la revendication 1, **caractérisée en ce que** la phase aqueuse comprend au moins un gélifiant.

4. Composition selon la revendication 1, **caractérisée en ce que** l'huile minérale est une huile de paraffine.

5. Composition selon la revendication 1, **caractérisée en ce que** le polyol est la glycérine.

6. Composition selon la revendication 1 **caractérisée en ce que** la composition comprend en outre un conservateur choisi parmi le methylparabène, propyl parabène, alcool benzylique et sorbate de potassium.

7. Composition selon l'une quelconque des revendications précédentes, comprenant:
- une phase grasse, comprenant entre 0,00001 et 1 % d'acide 3"-tert-butyl-4'-(2-hydroxyethoxy)-4"-pyrrolidin-1-yl[1,1'-3',1"]terphenyl-4-carboxylique,
- une phase aqueuse comprenant:
- de 0.1 à 15% d'esters de sucrose,
- de 1 à 40% de polyol,
- de 0.005 à 10% de gélifiant hydrophile,
- au moins 5% d'eau,
- de 0 à 15% d'un ou plusieurs additifs.

8. Composition selon la revendication 7 **caractérisée en ce que** la phase grasse est composée en outre de:
- 1% de phénoxyéthanol;
- 1,980% d'alcool cétearylique;
- 3% d'huiles minérales;
- et 15 à 22% d'huiles co-solvantes.

9. Composition selon l'une quelconque des revendications précédentes, comprenant:
- une phase grasse comprenant de 0.00001 % à 1 % d'acide 3"-tert-butyl-4'-(2-hydroxyethoxy)-4"-pyrrolidin-1-yl[1,1';3',1"]terphenyl-4-carboxylique; de 0.1 à 10% de phénoxyéthanol en tant que solvant principal du composé A; de 0.5 à 60% d'huiles co-solvantes du composé A; de 0.5 à 20% d'huiles minérales; de 0.1 à 10%, de préférence de 0.5 à 3 % d'épaississant de phase grasse; de 0 à 20% d'huile de silicone;
- une phase aqueuse comprenant de 0.1 à 15% d'émulsionnants de la famille des esters sucrose; de 1 à 40%, de préférence de 4 à 10% de polyol; de 0,005 à 10 % de gélifiant de la phase aqueuse;
- de 0.01 à 5% de système conservateur;
- et de 0 à 15% de 0.1 à 10% d'additifs.

10. Composition pour le traitement de l'acné composée:
- d'une phase grasse comprenant de 0.00001 % à 1 % d'acide 3"-tert-butyl-4'-(2-hydroxyéthoxy)-4"-pyrrolidin-1-yl[1,1';3',1"]terphényl-4-carboxylique (composé A); de 0.2 à 5% de phénoxyéthanol en tant que solvant principal du composé A; de 0.5 à 60% d'huiles co-solvantes du composé A choisies parmi Caprylic/ capric triglycérides, l'huile d'amande douce. Apricot Kernel Oil PEG-6 Ester, l'huile de coprah raffiné: de 0.5 à 20% d'huiles minérales; de 0.1 à 10% d'épaississant de phase grasse; de 0 à 20% d'huile de silicone;
- d'une phase aqueuse comprenant de 0.1 à 15% d'émulsionnants de la famille des esters sucrose; de 1 à 40% de polyol; de 0,005 à 10 % de gélifiant de la phase aqueuse;
- de 0.01 à 5% de système conservateur choisi parmi le methylparabène, le propylparabène, l'alcool benzylique et le sorbate de potassium;
- et de 0.001 à 15% d'additifs.

11. Composition pour le traitement des ichtyoses, les états ichtyosiformes, l'hyperkératose palmoplantaire ou le psoriasis composée:
- d'une phase grasse comprenant de 0.00001% à 1% d'acide 3"-tert-butyl-4'-(2-hydroxyéthoxy)-4"-pyrrolidin-1-yl[1,1';3',1"]terphényl-4-carboxylique (composé A); de 0.2 à 5% de phénoxyéthanol en tant que solvant principal du composé A; de 0.5 à 60% d'huiles co-solvantes du composé A, choisies parmi caprylic/capric triglycérides, l'huile d'amande douce, Apricot Kernel Oil PEG-6 Ester, l'huile de coprah raffiné; de 0.5 à 20% d'huiles minérales ; de 0.1 à 10% d'épaississant de phase grasse; de 0 à 20% d'huile de silicone;
- d'une phase aqueuse comprenant de 0.1 à 15% d'émulsionnants de la famille des esters de sucrose; de 1 à 40% de polyol; de 0,005 à 10 % de gélifiant de la phase aqueuse;
- de 0.01 à 5% de système conservateur choisi parmi le methylparabène, le propylparabène, l'alcool benzylique, et le sorbate de potassium:
- et de 0.001 à 15% d'additifs.

12. Composition pharmaceutique selon l'un quelque des revendications 1 à 9 pour son utilisation dans le traitement de pathologies suivantes:
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal);
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides;
5) Toute condition liée à des proliférations dermiques ou épidermiques bénignes, qu'elles soient ou non d'origine virale telles que verrues vulgaires, les verrues planes, le molluscum contagiosum et l'épidermodysplasie verruciforme, les papillomatoses orales ou florides;
6) les désordres dermatologiques tels que les dermatoses immunes comme le lupus érythémateux, les maladies immunes bulleuses et les maladies du collagène, telle la sclérodermie;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) les affections d'origine fongique au niveau cutané tel que le tinea pedis et le tinea versicolor,
10) les désordres de la pigmentation, tel l'hyperpigmentation, le mélasma, l'hypopigmentation ou le vitiligo;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tek que le lymphome

13. Composition pharmaceutique selon l'une quelconque des revendications 1 à 9 pour son utilisation selon la revendication 12, **caractérisée en ce que** la pathologie traitée est l'acné.

## Patentansprüche

1. Pharmazeutische Zusammensetzung vom Typ Öl-in-Wasser-Emulsion, umfassend:
- eine Fettphase, die 3"-tert-Butyl-4'-(2-hydroxyethoxy)-4"-pyrrolidin-1-yl[1,1';3',1"]terphenyl-4-carbonsäure (Verbindung A) als Wirkstoff enthält, Phenoxyethanol als Hauptlösungsmittel der Verbindung A, ein oder mehrere Co-Lösungsmittelöle, ausgewählt aus Capryl/Caprintriglyceriden, Süßmandelöl, Aprikosenkernöl PEG-6 Ester, raffiniertem Kokosnussöl und einem oder mehreren Mineralölen,
- eine wässrige Phase, die mindestens ein Tensid aus der Familie der Saccharoseester, mindestens ein Polyol und gereinigtes Wasser umfasst.

2. Zusammensetzung nach Anspruch 1, **dadurch gekennzeichnet, dass** das Tensid aus Saccharosepalmitat oder Saccharosestearat oder einer Mischung davon ausgewählt ist.

3. Zusammensetzung nach Anspruch 1, **dadurch gekennzeichnet, dass** die wässrige Phase mindestens ein Geliermittel umfasst.

4. Zusammensetzung nach Anspruch 1, **dadurch gekennzeichnet, dass** das Mineralöl ein Paraffinöl ist.

5. Zusammensetzung nach Anspruch 1, **dadurch gekennzeichnet, dass** das Polyol ein Glycerin ist.

6. Zusammensetzung nach Anspruch 1, **dadurch gekennzeichnet, dass** die Zusammensetzung ferner ein Konservierungsmittel, ausgewählt aus Methylparaben, Propylparaben, Benzylalkohol und Kaliumsorbat, umfasst.

7. Zusammensetzung nach einem der vorhergehenden Ansprüche, umfassend:
- eine Fettphase, die zwischen 0,00001 und 1 % 3"-tert-Butyl-4'-(2-hydroxyethoxy)-4"-pyrrolidin-1-yl[1,1';3',1"]terphenyl-4-carbonsäure umfasst,
- eine wässrige Phase umfassend:
- 0,1 bis 15 % Saccharoseester,
- 1 bis 40 % Polyol,
- 0,005 bis 10 % hydrophiles Geliermittel,
- mindestens 5 % Wasser,
- 0 bis 15 % einer oder mehrere Zusatzstoffe.

8. Zusammensetzung nach Anspruch 7, **dadurch gekennzeichnet, dass** die Fettphase ferner Folgendes umfasst:
- 1 % Phenoxyethanol;
- 1,980% Cetearylalkohol;
- 3 % Mineralöle;
- und 15 bis 22 % Co-Lösungsmittelöle.

9. Zusammensetzung nach einem der vorhergehenden Ansprüche, umfassend:
- eine Fettphase, umfassend 0,00001 % bis 1 % 3"-tert-Butyl-4'-(2-hydroxyethoxy)-4"-pyrrolidin-1-yl[1,1';3',1"]terphenyl-4-carbonsäure; 0,1 bis 10 % Phenoxyethanol als Hauptlösungsmittel der Verbindung A; 0,5 bis 60 % Co-Lösungsmittelöle der Verbindung A; 0,5 bis 20 % Mineralöle; 0,1 bis 10 %, vorzugsweise 0,5 bis 3 % Fettphasenverdickungsmittel; 0 bis 20 % Silikonöl;
- eine wässrige Phase, die 0,1 bis 15 % Emulgatoren aus der Familie der Saccharoseester; 1 bis 40 %, vorzugsweise 4 bis 10 % Polyol; 0,005 bis 10 % Geliermittel der wässrigen Phase enthält;
- 0,01 bis 5 % Konservierungssystem;
- und 0 bis 15 % von 0,1 bis 10 % Zusatzstoffe.

10. Zusammensetzung zur Behandlung von Akne, enthaltend:
- eine Fettphase, umfassend 0,00001 % bis 1 % 3"-tert-Butyl-4'-(2-hydroxyethoxy)-4"-pyrrolidin-1-yl[1,1';3',1"]terphenyl-4-carbonsäure (Verbindung A); 0,2 bis 5 % Phenoxyethanol als Hauptlösungsmittel der Verbindung A; 0,5 bis 60 % Co-Lösungsmittelöle der Verbindung A, ausgewählt aus Capryl/Caprintriglyceriden, Süßmandelöl, Aprikosenkernöl PEG-6 Ester, raffiniertes Kokosnussöl: 0,5 bis 20 % Mineralöle; 0,1 bis 10 % Fettphasenverdicker; 0 bis 20 % Silikonöl;
- eine wässrige Phase umfassend 0,1 bis 15 % Emulgatoren aus der Familie der Saccharoseester; 1 bis 40 % Polyol; 0,005 bis 10 % Geliermittel der wässrigen Phase;
- 0,01 bis 5 % Konservierungssystem, ausgewählt aus Methylparaben, Propylparaben, Benzylalkohol und Kaliumsorbat;
- und 0,001 bis 15 % Zusatzstoffe.

11. Zusammensetzung zur Behandlung von Ichthyose, ichthyosiformen Zuständen, palmoplantarer Hyperkeratose oder Psoriasis, enthaltend:
- eine Fettphase, umfassend 0,00001 % bis 1 % 3"-tert-Butyl-4'-(2-hydroxyethoxy)-4"-pyrrolidin-1-yl[1,1';3',1"]terphenyl-4-carbonsäure (Verbindung A); 0,2 bis 5 % Phenoxyethanol als Hauplösungsmittel del Verbindung A; 0,5 bis 60 % Co-Lösungsmittelöle der Verbindung A, ausgewählt aus Capryl/Caprintriglyceriden, Süßmandelöl, Aprikosenkernöl PEG-6 Ester, raffiniertes Kokosnussöl: 0,5 bis 20 % Mineralöle; 0,1 bis 10 % Fettphasenverdicker; 0 bis 20 % Silikonöl;
- eine wässrige Phase umfassend 0,1 bis 15 % Emulgatoren aus der Familie der Saccharoseester; 1 bis 40 % Polyol; 0,005 bis 10 % Geliermittel der wässrigen Phase;
- 0,01 bis 5 % Konservierungssystem, ausgewählt aus Methylparaben, Propylparaben, Benzylalkohol und Kaliumsorbat;
- und 0,001 bis 15 % Zusatzstoffe.

12. Pharmazeutische Zusammensetzung nach einem der Ansprüche 1 bis 9 zur Verwendung bei der Behandlung folgender Krankheiten:
1) dermatologische Erkrankungen, die mit einer Keratinisierungsstörung verbunden sind, die die Zelldifferenzierung und -proliferation betrifft, insbesondere zur Behandlung von Akne vulgaris, Komedonen, polymorpher Akne, Rosacea, nodulozystischer Akne, Akne conglobata, Altersakne, sekundärer Akne wie sonnenbedingte, medikamentös bedingte oder berufsbedingte Akne;
2) Verhornungsstörungen, insbesondere Ichthyose, ichthyosiforme Zustände, Iamelläre Ichthyose, Darier-Krankheit, palmoplantare Keratodermie, Leukoplakie, Pityriasis rubra pilaris und leukoplasiforme Zustände, kutane oder mukosale (orale) Flechte;
3) dermatologische Erkrankungen mit einer entzündlichen immun-allergischen Komponente, mit oder ohne Störung der Zellproliferation, insbesondere alle Formen von Psoriasis, auf der Haut, der Schleimhaut oder den Nägeln, oder sogar Psoriasis-Arthritis, oder die atopische Dermatitis und die verschiedenen Formen von Ekzemen;
4) Hautstörungen, die durch UV-Strahlung verursacht werden, sowie zur Behebung oder Bekämpfung der durch Licht verursachten oder chronologischen Hautalterung oder zur Verringerung von Pigmentierung und aktinischen Keratosen oder von Krankheiten, die mit chronologischer oder aktinischer Alterung einhergehen, wie Xerose, Pigmentierung und Falten;
5) alle Erkrankungen im Zusammenhang mit gutartigen dermalen oder epidermalen Wucherungen, unabhängig davon, ob sie viralen Ursprungs sind oder nicht, wie gewöhnliche Warzen, Flachwarzen, Molluscum contagiosum und Epidermodysplasia verruciformis, orale oder floride Papillomatose;
6) dermatologische Störungen wie immunvermittelte Dermatosen wie Lupus erythematodes, bullöse Autoimmunerkrankungen und Kollagenerkrankungen wie Sklerodermie;
7) Stigmata epidermaler und/oder dermaler Atrophie, die durch lokale oder systemische Corticosteroide induziert werden, oder jede andere Form der Hautatrophie,
8) Wundheilungsstörungen oder zur Vorbeugung oder Behebung von Dehnungsstreifen oder zur Förderung der Wundheilung,
9) Pilzerkrankungen der Haut wie Tinea pedis und Tinea versicolor,
10) Pigmentierungsstörungen wie Hyperpigmentierung, Melasma, Hypopigmentierung oder Vitiligo
11) krebsartige oder präkanzeröse Stufen der Haut oder der Schleimhaut wie aktinische Keratosen, Morbus Bowen, in-situ-Karzinome, Keratoakanthome und Hautkrebserkrankungen, wie Basalzellkarzinom (BCC), spinozelluläres Karzinom (SCC) und kutane Lymphome wie Lymphom

13. Pharmazeutische Zusammensetzung nach einem der Ansprüche 1 bis 9 zur Verwendung nach Anspruch 12, **dadurch gekennzeichnet, dass** die zu behandelnde Pathologie Akne ist.

## Claims

1. Oil-in-water emulsion-type pharmaceutical composition comprising:
- a fatty phase containing 3"-tert-butyl-4'-(2-hydroxyethoxy)-4"-pyrrolidin-1-yl[1,1';3',1"]terphenyl-4-carboxylic acid (compound A) as active ingredient, phenoxyethanol as the main solvent of compound A, one or more co-solvent oils selected from caprylic/capric triglycerides, sweet almond oil, Apricot Kernel Oil PEG-6 Ester, refined coconut oil, and one or more mineral oils,
- an aqueous phase comprising at least one surfactant from the sucrose ester family, at least one polyol and purified water.

2. Composition according to claim 1, **characterised in that** the surfactant is selected from sucrose palmitate, or sucrose stearate or a mixture thereof.

3. Composition according to claim 1, **characterised in that** the aqueous phase comprises at least one gelling agent.

4. Composition according to claim 1, **characterised in that** the mineral oil is a paraffin oil.

5. Composition of claim 1, **characterised in that** the polyol is glycerin.

6. Composition according to claim 1 **characterised in that** the composition further comprises a preservative selected from methylparaben, propylparaben, benzyl alcohol and potassium sorbate.

7. Composition according to any of the preceding claims, comprising:
- a fatty phase, comprising between 0.00001 and 1% 3"-tert-butyl-4'-(2-hydroxyethoxy)-4"-pyrrolidin-1-yl[1,1';3',1"]terphenyl-4-carboxylic acid,
- an aqueous phase comprising:
- 0.1 to 15% sucrose esters,
- 1 to 40% polyol,
- 0.005 to 10% hydrophilic gelling agent,
- at least 5% water,
- 0 to 15% one or more additives.

8. Composition according to claim 7, **characterised in that** the fatty phase is further comprised of:
- 1% phenoxyethanol;
- 1.980% cetearyl alcohol;
- 3% mineral oils;
- and 15 to 22% co-solvent oils.

9. Composition according to any of the preceding claims, comprising:
- a fatty phase comprising 0.00001% to 1% 3"-tert-butyl-4'-(2- hydroxyethoxy)-4"-pyrrolidin-1-yl[1,1';3',1"]terphenyl-4-carboxylic acid; 0.1 to 10% phenoxyethanol as the main solvent of compound A; 0.5 to 60% co-solvent oils of compound A; 0.5 to 20% mineral oils; 0.1 to 10%, preferably 0.5 to 3% fatty phase thickener; 0 to 20% silicone oil;
- an aqueous phase comprising 0.1 to 15% emulsifiers of the sucrose ester family; 1 to 40%, preferably 4 to 10% polyol; 0.005 to 10% gelling agent of the aqueous phase;
- 0.01 to 5% preservative system;
- and 0 to 15% of 0.1 to 10% additives.

10. Composition for the treatment of acne comprised of:
- a fatty phase comprising 0.00001% to 1% 3"-tert-butyl-4'-(2-hydroxyethoxy)-4"-pyrrolidin-1-yl[1,1';3',1"]terphenyl-4-carboxylic acid (compound A); 0.2 to 5% phenoxyethanol as the main solvent of compound A; 0.5 to 60% co-solvent oils of compound A selected from Caprylic/capric triglycerides, sweet almond oil, Apricot Kernel Oil PEG-6 Ester, refined coconut oil: 0.5 to 20% mineral oil; 0.1 to 10% fatty phase thickener; 0 to 20% silicone oil;
- an aqueous phase comprising 0.1 to 15% emulsifiers of the sucrose ester family; 1 to 40% polyol; 0.005 to 10% gelling agent of the aqueous phase;
- 0.01 to 5% preservative system selected from methylparaben, propylparaben, benzyl alcohol and potassium sorbate;
- and 0.001 to 15% additives.

11. Composition for the treatment of ichthyoses, ichthyosiform conditions, palmoplantar hyperkeratosis or psoriasis, comprised of:
- a fatty phase comprising 0.00001% to 1% 3"-tert-butyl-4'-(2-hydroxyethoxy)-4"-pyrrolidin-1-yl[1,1';3',1"]terphenyl-4-carboxylic acid (compound A); 0.2 to 5% phenoxyethanol as the main solvent for compound A; 0.5 to 60% co-solvent oils of compound A, selected from caprylic/capric triglycerides, sweet almond oil, Apricot Kernel Oil PEG-6 Ester, refined coconut oil; 0.5 to 20% mineral oils; 0. to 10% fatty phase thickener; 0 to 20% silicone oil;
- an aqueous phase comprising 0.1 to 15% emulsifiers of the sucrose ester family; 1 to 40% polyol; 0.005 to 10% gelling agent of the aqueous phase
- 0.01 to 5% preservative system selected from methylparaben, propylparaben, benzyl alcohol and potassium sorbate:
- and 0.001 to 15% additives.

12. Pharmaceutical composition according to any of claims 1 to 9 for use in the treatment of the following pathologies:
1) dermatological conditions linked to a keratinization disorder concerning cellular differentiation and proliferation, in particular for treating acne vulgaris, comedonal acne, polymorphic acne, acne rosacea, nodulocystic acne, acne conglobata, senile acne, secondary acne such as sun-induced, drug-induced or occupational acne;
2) keratinisation disorders, in particular ichthyoses, ichthyosiform states, lamellar ichthyosis, Darier disease, palmoplantar keratoderma, leukoplakia, pityriasis rubra pilaris and leukoplakiform conditions, cutaneous or mucosal (oral) lichen;
3) dermatological conditions with an inflammatory immuno-allergic component, with or without cell proliferation disorder, and in particular all forms of psoriasis, whether cutaneous, mucous or ungual, and even psoriatic arthritis, or atopic dermatitis and the various forms of eczema;
4) skin disorders due to exposure to UV radiation, as well as for repairing or combatting skin ageing, whether photo-induced or chronological, or for reducing pigmentation and actinic keratoses, or any pathologies associated with chronological or actinic ageing, such as xerosis, pigmentation and wrinkles;
5) any condition related to benign dermal or epidermal proliferations, whether or not of viral origin, such as common warts, flat warts, molluscum contagiosum and epidermodysplasia verruciformis, oral or florid papillomatosis;
6) dermatological disorders such as immune-mediated dermatoses like lupus erythematosus, autoimmune blistering disorders and collagen diseases such as scleroderma;
7) stigmata from epidermal and/or dermal atrophy induced by local or systemic corticosteroids, or any other form of skin atrophy
8) wound healing disorders, or for preventing or repairing stretch marks, or for promoting wound healing,
9) fungal skin diseases such as tinea pedis and tinea versicolor,
10) pigmentation disorders such as hyperpigmentation, melasma, hypopigmentation or vitiligo
11) cancerous or precancerous conditions of the skin or the mucous membranes such as actinic keratoses, Bowen's disease, carcinoma *in situ*, keratoacanthoma and skin cancers such as basal cell carcinoma (BCC), squamous cell carcinoma (SCC) and skin lymphomas such as lymphoma

13. Pharmaceutical composition according to any of claims 1 to 9 for use according to claim 12, **characterised in that** the pathology to be treated is acne.
